# EUROPEAN PATENT APPLICATION

(11) **EP 4 360 704 A2**
(43) Date of publication of application: **01.05.2024**
(21) Application number: 24150717.7
(22) Date of filing: 29.09.2016
(51) Int. Cl.: A61P 3/10

(54) **METHOD OF TREATING MELANOCORTIN-4 RECEPTOR PATHWAY-ASSOCIATED DISORDERS**

(30) Priority: 30.09.2015 US 201562235003 P
(62) Divisional of application: 16782150.3
(71) Applicant: Rhythm Pharmaceuticals, Inc., Boston, MA 02116 (US)
(72) Inventor: SHARMA, Shubh, Cranbury, NJ 08512 (US); VAN DER PLOEG, Leonardus H.T., Newton, MA 02467 (US); HENDERSON, Bart, Belmont, MA 02478 (US)
(74) Representative: J A Kemp LLP

(57) **Abstract**

The disclosure is related to a method of treating a disorder, such as obesity or a condition associated with obesity, such as hyperphagia, in a subject using a melanocortin-4 receptor (MC4R) agonist.

## Description

### BACKGROUND OF THE INVENTION

Melanocortin 4 receptor (MC4R) is a heterotrimeric G-protein-coupled receptor, which transduces signals by activating second messenger systems, including adenylate cyclase. Expressed in hypothalamic nuclei and other neuronal and non-neuronal tissues, controlling feeding behavior and energy homeostasis, MC4R integrates an agonist (anorexigenic) signal provided by the α-melanocyte stimulating hormone (α-MSH), and an antagonist (orexigenic) signal provided by the agouti-related peptide (AGPR). Signals from MC4R modulate feeding behavior through secondary effector neurons.

### SUMMARY OF THE INVENTION

The invention is based in part on the discovery of novel MC4R agonists. They are useful, e.g., in treating disorders associated with obesity. Obesity disorders include, e.g., polygenic and monogenic obesity disorders, including morbid obesity. Treatment of obesity results in reduced body weight mainly due to a loss of fat mass often due to pharmacotherapy associated with a reduction in appetite and an increase or normalization of energy expenditure. Improvements in comorbidities can include reduced insulin resistance and improvement in cardiovascular parameters, as a result of reduced blood pressure and/or heart rate.

Accordingly, in one aspect, the invention features a compound of Formula I or II (or a pharmaceutically acceptable salt thereof). Formula I has the structure set forth below. 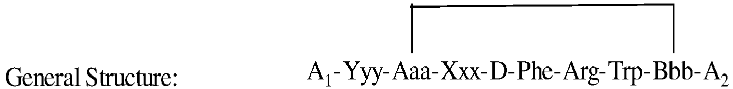
Aaa, Bbb = Selected from Cys, hCys, Pen; capable of establishing a disulfide bridge or
   Glu, Asp, Lys, Orn, Dpr, Dbu capable of establishing a lactam bridge
Xxx = Asn, Gln, Ser, Thr
Yyy = Lys, Arg, D-Lys, D-Arg
A₁ = H, Ac
A₂ = OH, NH₂
Ac is acyl, e.g., acetyl.

In embodiments, the MC4R agonist is a compound of the following structure (Formula II) or a pharmaceutically acceptable salt thereof: 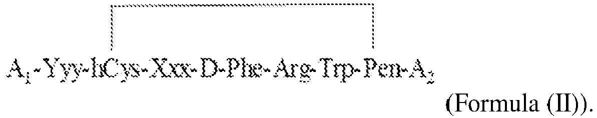
where Xxx is Asn, Gln, Ser, or Thr,
where A₁ is H or Ac,
where A₂ is OH or NH₂, and
where Yyy is Lys, Arg, D-Lys, or D-Arg.

In embodiments, the MC4R agonist is chosen from one or more of the following compounds, (or pharmaceutically acceptable salt thereof):
001554C Ac-Arg-(hCys-Asn-D-Phe-Arg-Trp-Pen)-NH₂ (SEQ ID NO: 1)
001555C Ac-Arg-(hCys-Gln-D-Phe-Arg-Trp-Pen)-NH₂ (SEQ ID NO: 2)
001556C Ac-Arg-(hCys-Ser-D-Phe-Arg-Trp-Pen)-NH₂ (SEQ ID NO: 3)
001574C: Ac-Arg-(hCys-Thr-D-Phe-Arg-Trp-Pen)-NH₂ (SEQ ID NO: 4)
001576C: Ac-Arg-(Glu-Gln-D-Phe-Arg-Trp-Apr)-NH₂ (SEQ ID NO: 5)
Ac-Arg-(hCys-Asn-D-Phe-Arg-Trp-Pen)-OH (SEQ ID NO: 6)
H-Arg-(hCys-Asn-D-Phe-Arg-Trp-Pen)-NH₂ (SEQ ID NO: 7)
H-Arg-(hCys-Asn-D-Phe-Arg-Trp-Pen)-OH (SEQ ID NO: 8)
Ac-D-Arg-(hCys-Asn-D-Phe-Arg-Trp-Pen)-NH₂ (SEQ ID NO: 9)
H-D-Arg-(hCys-Asn-D-Phe-Arg-Trp-Pen)-NH₂ (SEQ ID NO: 10)
Ac-D-Arg-(hCys-Asn-D-Phe-Arg-Trp-Pen)-OH (SEQ ID NO:11)
H-D-Arg-(hCys-Asn-D-Phe-Arg-Trp-Pen)-OH (SEQ ID NO: 12)
Ac-Lys-(hCys-Asn-D-Phe-Arg-Trp-Pen)-NH₂ (SEQ ID NO: 13)
Ac-Lys-(hCys-Asn-D-Phe-Arg-Trp-Pen)-OH (SEQ ID NO: 14)
H-Lys-(hCys-Asn-D-Phe-Arg-Trp-Pen)-NH₂ (SEQ ID NO:15)
H-Lys-(hCys-Asn-D-Phe-Arg-Trp-Pen)-OH (SEQ ID NO: 16)
Ac-D-Lys-(hCys-Asn-D-Phe-Arg-Trp-Pen)-NH₂ (SEQ ID NO: 17)
H-D-Lys-(hCys-Asn-D-Phe-Arg-Trp-Pen)-NH₂ (SEQ ID NO: 18)
Ac-D-Lys-(hCys-Asn-D-Phe-Arg-Trp-Pen)-OH (SEQ ID NO: 19)
H-D-Lys-(hCys-Asn-D-Phe-Arg-Trp-Pen)-OH (SEQ ID NO: 20)
Ac-Arg-(hCys-Gln-D-Phe-Arg-Trp-Pen)-OH (SEQ ID NO: 21)
H-Arg-(hCys-Gln-D-Phe-Arg-Trp-Pen)-NH₂ (SEQ ID NO: 22)
H-Arg-(hCys-Gln-D-Phe-Arg-Trp-Pen)-OH (SEQ ID NO: 23)
Ac-D-Arg-(hCys-Gln-D-Phe-Arg-Trp-Pen)-NH₂ (SEQ ID NO: 24)
H-D-Arg-(hCys-Gln-D-Phe-Arg-Trp-Pen)-NH₂ (SEQ ID NO: 25)
Ac-D-Arg-(hCys-Gln-D-Phe-Arg-Trp-Pen)-OH (SEQ ID NO: 26)
H-D-Arg-(hCys-Gln-D-Phe-Arg-Trp-Pen)-OH (SEQ ID NO: 27)
Ac-Lys-(hCys-Gln-D-Phe-Arg-Trp-Pen)-NH₂ (SEQ ID NO: 28)
Ac-Lys-(hCys-Gln-D-Phe-Arg-Trp-Pen)-OH (SEQ ID NO: 29)
H-Lys-(hCys-Gln-D-Phe-Arg-Trp-Pen)-NH₂ (SEQ ID NO: 30)
H-Lys-(hCys-Gln-D-Phe-Arg-Trp-Pen)-OH (SEQ ID NO: 31)
Ac-D-Lys-(hCys-Gln-D-Phe-Arg-Trp-Pen)-NH₂ (SEQ ID NO: 32)
H-D-Lys-(hCys-Gln-D-Phe-Arg-Trp-Pen)-NH₂ (SEQ ID NO: 33)
Ac-D-Lys-(hCys-Gln-D-Phe-Arg-Trp-Pen)-OH (SEQ ID NO: 34)
H-D-Lys-(hCys-Gln-D-Phe-Arg-Trp-Pen)-OH (SEQ ID NO: 35)
Ac-Arg-(hCys-Ser-D-Phe-Arg-Trp-Pen)-OH (SEQ ID NO: 36)
H-Arg-(hCys-Ser-D-Phe-Arg-Trp-Pen)-NH₂ (SEQ ID NO: 37)
H-Arg-(hCys-Ser-D-Phe-Arg-Trp-Pen)-OH (SEQ ID NO: 38)
Ac-D-Arg-(hCys-Ser-D-Phe-Arg-Trp-Pen)-NH₂ (SEQ ID NO: 39)
H-D-Arg-(hCys-Ser-D-Phe-Arg-Trp-Pen)-NH₂ (SEQ ID NO: 40)
Ac-D-Arg-(hCys-Ser-D-Phe-Arg-Trp-Pen)-OH (SEQ ID NO:41)
H-D-Arg-(hCys-Ser-D-Phe-Arg-Trp-Pen)-OH (SEQ ID NO:42)
Ac-Lys-(hCys-Ser-D-Phe-Arg-Trp-Pen)-NH₂ (SEQ ID NO: 43)
Ac-Lys-(hCys-Ser-D-Phe-Arg-Trp-Pen)-OH (SEQ ID NO:44)
H-Lys-(hCys-Ser-D-Phe-Arg-Trp-Pen)-NH₂ (SEQ ID NO: 45)
H-Lys-(hCys-Ser-D-Phe-Arg-Trp-Pen)-OH (SEQ ID NO: 46)
Ac-D-Lys-(hCys-Ser-D-Phe-Arg-Trp-Pen)-NH₂ (SEQ ID NO: 47)
H-D-Lys-(hCys-Ser-D-Phe-Arg-Trp-Pen)-NH₂ (SEQ ID NO: 48)
Ac-D-Lys-(hCys-Ser-D-Phe-Arg-Trp-Pen)-OH (SEQ ID NO: 49)
H-D-Lys-(hCys-Ser-D-Phe-Arg-Trp-Pen)-OH (SEQ ID NO: 50)
Ac-Arg-(hCys-Thr-D-Phe-Arg-Trp-Pen)-OH (SEQ ID NO: 51)
H-Arg-(hCys-Thr-D-Phe-Arg-Trp-Pen)-NH₂ (SEQ ID NO: 52)
H-Arg-(hCys-Thr-D-Phe-Arg-Trp-Pen)-OH (SEQ ID NO: 53)
Ac-D-Arg-(hCys-Thr-D-Phe-Arg-Trp-Pen)-NH₂ (SEQ ID NO: 54)
H-D-Arg-(hCys-Thr-D-Phe-Arg-Trp-Pen)-NH₂ (SEQ ID NO: 55)
Ac-D-Arg-(hCys-Thr-D-Phe-Arg-Trp-Pen)-OH (SEQ ID NO: 56)
H-D-Arg-(hCys-Thr-D-Phe-Arg-Trp-Pen)-OH (SEQ ID NO: 57)
Ac-Lys-(hCys-Thr-D-Phe-Arg-Trp-Pen)-NH₂ (SEQ ID NO: 58)
Ac-Lys-(hCys-Thr-D-Phe-Arg-Trp-Pen)-OH (SEQ ID NO: 59)
H-Lys-(hCys-Thr-D-Phe-Arg-Trp-Pen)-NH₂ (SEQ ID NO: 60)
H-Lys-(hCys-Thr-D-Phe-Arg-Trp-Pen)-OH (SEQ ID NO: 61)
Ac-D-Lys-(hCys-Thr-D-Phe-Arg-Trp-Pen)-NH₂ (SEQ ID NO: 62)
H-D-Lys-(hCys-Thr-D-Phe-Arg-Trp-Pen)-NH₂ (SEQ ID NO: 63)
Ac-D-Lys-(hCys-Thr-D-Phe-Arg-Trp-Pen)-NH₂ (SEQ ID NO: 64) or
H-D-Lys-(hCys-Thr-D-Phe-Arg-Trp-Pen)-NH₂ (SEQ ID NO: 65).

In another aspect, the invention provides, a method of eliciting an agonist effect from a melanocortin receptor in a subject in need thereof which comprises administering to said subject an effective amount of a compound of formula (I) or formula (II) as defined hereinabove, or a pharmaceutically acceptable salt thereof.

In another aspect, the invention provides a method of treating a
condition or a disorder, e.g., a condition associated with obesity, by administering a compound of formula (I) or formula (II) as defined hereinabove, or a pharmaceutically acceptable salt thereof.

In another aspect, the invention provides a method of treating a metabolic disease or medical condition accompanied by weight gain such as obesity, feeding disorders and Prader-Willi Syndrome by administering an effective amount of a compound of formula (I) or formula (II) as defined hereinabove, or a pharmaceutically acceptable salt thereof. While not wishing to be bound by theory, it is believed that an agonist anti-obesity effect is elicited from the melanocortin receptor. In a further aspect of the foregoing method, the disease or condition treated is obesity. In yet a further aspect of the foregoing method, the disease or condition treated is a feeding disorder.

In another aspect, the present invention provides a method of decreasing food intake, decreasing body weight or a combination thereof, by administering an effective amount of a compound of formula (I) or formula (II) as defined hereinabove, or a pharmaceutically acceptable salt thereof. In a preferred embodiment, the present invention provides a method of decreasing food intake, decreasing body weight or a combination thereof, by eliciting an agonist or antagonist effect from a melanocortin receptor by administering an effective amount of a compound of formula (I) or (II) or a pharmaceutically acceptable salt thereof.

In another aspect, the present invention provides a method of decreasing appetite without compromising body weight by administering an effective amount of a compound of formula (I) or formula (II) as defined hereinabove, or a pharmaceutically acceptable salt thereof. In another aspect, the present invention, provides a method of decreasing food consumption while decreasing body weight by administering an effective amount of a compound of formula (I) or formula (II) as defined hereinabove, or a pharmaceutically acceptable salt thereof. In another aspect, the present invention provides a method for increasing or normalizing energy expenditure, e.g., facilitating and/or effecting weight loss due to a reduction in fat mass in the subject.

In embodiments, the condition or disorder is obesity, e.g., Prader-Willi Syndrome. In embodiments the agonist it administered to decrease food intake in a subject in need thereof, or to decrease body weight.

In an embodiment a method described herein comprises ameliorating or improving a clinical symptom or indicators associated with a disorder described herein such as obesity, type-II diabetes, pre-diabetic condition, blood level of haemoglobin A1C (HblAc) above 6%, hyperinsulimenia, hyperlipidemia, insulin insensitivity, or glucose intolerance; delaying, inhibiting or preventing the progression of obesity and/or obesity related indications; or partially or totally delaying, inhibiting or preventing the onset or development of obesity or a obesity related indication.

In embodiments, the method comprises administering the agonist in a unit dosage suitable for injection, e.g., subcutaneous injection, e.g., oral administration, suppository mediated administration or administration of the compound by absorption through the skin, intranasal administration or sublingual administration, to the subject.

In embodiments, the daily dosage is 0.1 mg to 10 mg. In embodiments, the daily dosage is about 0.1 mg to about 7.5 mg. In embodiments, the daily dosage is about 0.1 mg to about 5 mg. In embodiments, the daily dosage is about 0.1 mg to about 2.5 mg. In embodiments, the daily dosage is about 0.1 mg to about 2 mg. In embodiments, the daily dosage is about 0.1 mg to about 1 mg. In embodiments, the daily dosage is about 0.2 mg to about 10 mg. In embodiments, the daily dosage is about 0.2 mg to about 7.5 mg. In embodiments, the daily dosage is about 0.2 mg to about 5 mg. In embodiments, the daily dosage is about 0.2 mg to about 2.5 mg. In embodiments, the daily dosage is about 0.2 mg to about 2 mg. In embodiments, the daily dosage is about 0.2 mg to about 1.5 mg. In embodiments, the daily dosage is about 0.2 mg to about 1 mg. In embodiments, the daily dosage is about 0.3 mg to about 10 mg. In embodiments, the daily dosage is about 0.3 mg to about 7.5 mg. In embodiments, the daily dosage is about 0.3 mg to about 5 mg. In embodiments, the daily dosage is about 0.3 mg to about 2.5 mg. In embodiments, the daily dosage is about 0.3 mg to about 2 mg. In embodiments, the daily dosage is about 0.3 mg to about 1.5 mg. In embodiments, the daily dosage is about 0.3 mg to about 1 mg. In embodiments, the daily dosage is about 0.25 mg (e.g., 0.25 mg) to about 0.5 mg (e.g., 0.5 mg). In embodiments, the daily dosage is about 0.5 mg (e.g., 0.5 mg) to about 0.75 mg (e.g., 0.75 mg). In embodiments, the daily dosage is about 0.25 mg (e.g., 0.25 mg). In embodiments, the daily dosage is about 0.5 mg (e.g., 0.5 mg). In embodiments, the daily dosage is about 0.75 mg (e.g., 0.75 mg) to about 1.25 mg (1.25 mg). In embodiments, the daily dosage is about 1 mg (e.g., 1 mg). In embodiments, the daily dosage is about 1.25 mg (e.g., 1.25 mg) to about 2 mg (e.g., 2 mg). In embodiments, the daily dosage is about 1.5 mg (e.g., 1.5 mg). In embodiments, the daily dosage is about 2 mg (e.g., 2 mg).

In embodiments, the method comprises administering the agonist in a unit dosage suitable for injection, e.g., subcutaneous injection, to the subject.

In embodiments, the unit dosage comprises about 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, or 2 mg of the agonist.

In embodiments, the unit dosage is disposed within a delivery device, e.g., a syringe (e.g., prefilled syringe), an implantable device, a needleless hypodermic injection device, an infusion pump (e.g., implantable infusion pump), or an osmotic delivery system.

In embodiments, the agonist is administered subcutaneously, e.g., by subcutaneous injection.

In embodiments, the agonist is administered systemically e.g., by oral dosing or other route in a dose range, e.g., from 0.1 mg to 100 mg, from 1 mg to 500 mg, or from 10 mg to 1000 mg, to attain effective therapeutic concentrations.

In embodiments, the agonist is administered systemically e.g. by oral dosing or other route in a dose range form in a dose range from 0.1 mg to 100 mg, from 1 mg to 500 mg, from 10 mg to 1000 mg, to attain effective therapeutic concentrations.

In embodiments, the agonist is administered daily over a period of at least 3 weeks, e.g., at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 weeks or more, or at least 1, 2, 3, 4, 5, 6, 7, 8, 9, 10, 11, or 12 months or more, or at least 1, 2, 3, 4 years or more.

In embodiments, the subject is obese, e.g., severely obese.

In embodiments, the subject has early onset severe obesity.

In embodiments, the subject is hyperphagic.

In embodiments, the subject has a body mass index (BMI) greater than 25 kg/m² (e.g., ≥25, 30, 31, 32, 33, 34, 35, 36, 37, 38, 39, 40, 41, 42, 43, 44, 45, 46, 47, 48, 49, 50 kg/m² or greater) prior to administration of the agonist, e.g., at the time the agonist is prescribed, or at the time of the first administration.

In embodiments, the subject has a body mass index (BMI) greater than 35 kg/m² (e.g., ≥36, 37, 38, 39, 40, 41, 42, 43, 44, 45, 46, 47, 48, 49, 50 kg/m² or greater) prior to administration of the agonist, e.g., at the time the agonist is prescribed, or at the time of the first administration.

In embodiments, the subject has a body mass index (BMI) greater than 40 kg/m² (e.g., ≥41, 42, 43, 44, 45, 46, 47, 48, 49, 50, 51, 52, 53, 54, 55 kg/m² or greater) prior to administration of the agonist, e.g., at the time the agonist is prescribed, or at the time of the first administration.

In embodiments, the subject has a body mass index (BMI) greater than 45 kg/m² (e.g., ≥46, 47, 48, 49, 50, 51, 52, 53, 54, 55 kg/m² or greater) prior to administration of the agonist, e.g., at the time the agonist is prescribed, or at the time of the first administration.

In embodiments, the subject has a BMI higher than the 85-95th percentile prior to administration of the agonist, e.g., at the time the agonist is prescribed, or at the time of the first administration.

In embodiments, the subject has failed one or more previous therapies, e.g., exercise, diet, surgery, including banded gastroplasty (such as vertical banded gastroplastay) or bariatric surgery or behavioral therapies, prior to administration of the agonist, e.g., at the time the agonist is prescribed, or at the time of the first administration.

In embodiments, the subject has a lower body weight after administration of the agonist than before administration of the agonist.

In embodiments, administration of the agonist results in a reduction of weight in the subject compared to the weight of the subject before treatment of about 0.5 kg to 3 kg after 1 week of treatment, or about 1 kg to 6 kg after 2 weeks of treatment, or about 2 kg to 12 kg after 4 weeks of treatment, or about 4 kg to 24 kg after 8 weeks of treatment, or about 8 kg to 48 kg after 16 weeks of treatment.

In embodiments, administration of the agonist results in weight loss in the subject at a rate of about 0.5-1 kg/week, 1-2 kg/week, e.g., about 2 kg/week, e.g., over a period of 1-2 weeks of treatment or longer, 2-4 weeks of treatment or longer, 4-8 weeks of treatment or longer, 8-16 weeks of treatment or longer, 16-32 weeks or longer, or 32-64 weeks or longer.

In embodiments, administration of the agonist results in a reduction in hunger level (e.g., a lower score on the Likert hunger scale, e.g., a lower score by at least 1, 2, 3, 4, 5, 6, 7, 8, 9 or 10 points) in the subject compared to the hunger level of the subject before treatment, e.g., results in abolishment of hunger (e.g., a score of 0 on the Likert hunger scale) in the subject, e.g., after 1-2 weeks of treatment or longer, 2-4 weeks of treatment or longer, 4-8 weeks of treatment or longer, or 8-16 weeks of treatment or longer.

In embodiments, administration of the agonist results in no detectable/significant decrease in resting energy expenditure (REE) in the subject, e.g., over a period of 24 hours, one week, or 30 days or longer, e.g., as compared to a control REE (e.g., the REE in the subject prior to treatment or a predetermined REE, e.g., in subjects of similar pre-treatment BMI, e.g., when expressed as REE per kg of lean body mass).

In embodiments, administration of the agonist results in an increase in resting energy expenditure (REE) in the subject, e.g., over a period of 24 hours, one week, or 30 days, or longer e.g., as compared to a control REE (e.g., the REE in the subject prior to treatment or compared to a predetermined REE, e.g., in subjects of similar pre-treatment BMI, when expressed as REE per kg of lean body mass, e.g., after a similar level of weight loss has been attained by fasting).

In embodiments, administration of the agonist results in a reduction in food intake by the subject compared to a control (e.g., the food intake of the subject prior to treatment), e.g., wherein the food intake is daily food intake or food intake over a period of 24 hours, or one week.

In embodiments, administration of the agonist results in a reduction in food intake of at least 100 kilocalories, e.g., at least 100, 125, 150, 175, 200, 225, 250, 275, 300, 325, 350, 375, 400, 425, 450, 475, 500, 525, 550, 575, 600, 1000 kilocalories or more, compared to a control (e.g., the food intake of the subject prior to treatment or a predetermined food intake level), e.g., wherein the food intake is daily food intake or food intake over a period of 24, hours, or one week.

In embodiments, administration of the agonist results in a reduction in food intake of at least 5 kcal/kg/day, e.g., 5, 10, 20, 30, 40, 50, 60, 70, 80, or 90 or more kcal/kg/day. In embodiments, the reduction in food intake is relative to the food intake at baseline. In embodiments, the baseline food intake is at least 100 kcal/kg/day, e.g., for a pediatric subject at about 1 year of age. In embodiments, the baseline food intake is at least 40 kcal/kg/day, e.g., for a pediatric subject, e.g., in late adolescence.

In embodiments, administration of the agonist results in a reduction in waist circumference of the subject compared to a control (e.g., the waist circumference of the subject prior to treatment), as measured 1, 2, 3, 4, 5, 6, 7, 8, 9, 10 weeks or more after initiation of treatment.

In embodiments, administration of the agonist results in a reduction in waist circumference of at least 2 cm (e.g., at least 2, 3, 4, 5, 6, 7, 8, 9, 10 cm or more) in the subject compared to a control (e.g., the waist circumference of the subject prior to treatment), as measured 1, 2, 3, 4, 5, 6, 7, 8, 9, 10 weeks or more after initiation of treatment.

In embodiments, administration of the agonist results in no detectable increase in blood pressure (e.g., diastolic and/or systolic blood pressure) of the subject compared to the blood pressure of the subject prior to treatment, as measured 1, 2, 3, 4, 5, 6, 7, 8, 9, 10 weeks or more after initiation of treatment.

In embodiments, administration of the agonist results in a reduction in blood pressure (e.g., diastolic and/or systolic blood pressure) of the subject compared to the blood pressure of the subject prior to treatment, as measured 1, 2, 3, 4, 5, 6, 7, 8, 9, 10 weeks or more after initiation of treatment.

In embodiments, administration of the agonist results in a reduction in systolic blood pressure of the subject of at least 1-3 mmHg (e.g., at least 1, 2, 3, 3.5, 4, 4.5, 5, 5.5, 6, 6.5, 7 mmHg or more) compared to the blood pressure of the subject prior to treatment, as measured 1, 2, 3, 4, 5, 6, 7, 8, 9, 10 weeks or more after initiation of treatment.

In embodiments, administration of the agonist results in a reduction in diastolic blood pressure of the subject of at least 4 mmHg (e.g., at least 4, 7, 7.5, 8, 8.5, 9, 9.5, 10 mmHg or more) compared to the blood pressure of the subject prior to treatment, as measured 1, 2, 3, 4, 5, 6, 7, 8, 9, 10 weeks or more after initiation of treatment.

In embodiments, the subject is a mammal, e.g., a human.

In an embodiment, the agonist is delivered by way of an implantable device, a needleless hypodermic injection device, an infusion pump (e.g., implantable infusion pump), or an osmotic delivery system.

In embodiments, the agonist is administered subcutaneously, e.g., by subcutaneous injection.

In an aspect, provided herein is a unit dosage of an agonist described herein, wherein the unit dosage contains 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.5, 3, 3.5, 4, 4.5, or 5 mg of the agonist.

In embodiments, the unit dosage contains 0.5 mg of agonist.

In embodiments, the unit dosage contains 1.0 mg of agonist.

In embodiments, the unit dosage contains 1.5 mg of agonist.

In embodiments, the unit dosage is suitable for injection, e.g., subcutaneous injection.

In embodiments, the unit dosage is disposed in a delivery device suitable for injection, e.g., subcutaneous injection.

In embodiments, the unit dosage is disposed in a syringe or pen-type injector suitable for injection, e.g., subcutaneous injection.

In accordance with any method described herein, in embodiments, an improved symptoms comprise one or more of:
(a) a decrease in body weight;
(b) a decrease in waist circumference;
(c) a decrease in hunger level;
(d) a decrease in food intake level;
(e) a lack of decrease or an increase in resting energy expenditure;
(f) a decrease in insulin resistance;
(g) a decrease in sleep apnea; or
(h) a decrease in symptoms associated with the metabolic syndrome.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In addition, the materials, methods, and examples are illustrative only and not intended to be limiting. Headings, subheadings or numbered or lettered elements, e.g., (a), (b), (i) etc, are presented merely for ease of reading. The use of headings or numbered or lettered elements in this document does not require the steps or elements be performed in alphabetical order or that the steps or elements are necessarily discrete from one another. Other features, objects, and advantages of the invention will be apparent from the description and drawings, and from the claims.

### DETAILED DESCRIPTION OF THE INVENTION

### Definitions

As used herein "about" and "approximately" generally mean an acceptable degree of error for the quantity measured given the nature or precision of the measurements. Exemplary degrees of error are within 20 percent (%), typically, within 10%, and more typically, within 5% of a given value or range of values.

"Acquire" or "acquiring" as the terms are used herein, refer to obtaining possession of a physical entity, or a value, *e.g.,* a numerical value, or knowledge of (e.g., knowledge of the sequence or mutational state of) a genotype or a nucleic acid or polypeptide, by "directly acquiring" or "indirectly acquiring" the physical entity, value, or knowledge. "Directly acquiring" means performing a physical process (*e.g*., performing a synthetic or analytical method) to obtain the physical entity, value, or knowledge. "Indirectly acquiring" refers to receiving the physical entity, value, or knowledge from another party or source (*e.g.,* a third party laboratory that directly acquired the physical entity, value, or knowledge). Directly acquiring a physical entity includes performing a process that includes a physical change in a physical substance, *e.g*., a starting material. Exemplary changes include making a physical entity from two or more starting materials, shearing or fragmenting a substance, separating or purifying a substance, combining two or more separate entities into a mixture, performing a chemical reaction that includes breaking or forming a covalent or non-covalent bond. Directly acquiring a value or knowledge includes performing a process that includes a physical change in a sample or another substance. Examples include performing an analytical process which includes a physical change in a substance, *e.g*., a sample, analyte, or reagent (sometimes referred to herein as "physical analysis"), performing an analytical method, *e.g.,* a method which includes one or more of the following: separating or purifying a substance, e.g., an analyte, or a fragment or other derivative thereof, from another substance; combining an analyte, or fragment or other derivative thereof, with another substance, e.g., a buffer, solvent, or reactant; or changing the structure of an analyte, or a fragment or other derivative thereof, *e.g.,* by breaking or forming a covalent or non-covalent bond, between a first and a second atom of the analyte; or by changing the structure of a reagent, or a fragment or other derivative thereof, *e.g.,* by breaking or forming a covalent or non-covalent bond, between a first and a second atom of the reagent.

As used herein, the term "obese" refers to a subject having a body mass index (BMI) within the ranges defined as "obese" by the Center for Disease Control (*see,* e.g., URL.cdc.gov/obesity/defining.html and www.cdc.gov/obesity/childhood/defining.html, last accessed on August 19, 2015) or as defined by "Clinical Guidelines on the Identification, Evaluation, and Treatment of Overweight and Obesity in Adults" from the National Institutes of Health. BMI is obtained by dividing a subject's weight, e.g., in kilograms (kg) by the square of the subject's height, e.g., in meter (m). For example, an adult who has a BMI of 30 kg/m² or higher is considered obese. For example, an adult with a BMI of 25.0 to 29.9 kg/m² is considered overweight; an adult with a BMI of 18.5 to 24.9 kg/m² is considered to have a normal or healthy weight range; and an adult with a BMI of less than 18.5 kg/m² is considered to be underweight. For example, an adult having a height of 5 feet, 9 inches with a body weight of 203 pounds or more is considered obese. For children and teens, obese refers to a subject having a BMI at or above the 85^{th} to 95^{th} percentile for children and teens of the same age and sex.

A "severely obese" subject or a subject having "severe obesity" refers to a subject having a BMI of 35 kg/m² or higher, e.g., 40 kg/m² or higher. For example, a severely obese subject is over 100% over the ideal (normal, healthy) body weight.

As used herein "early onset", e.g., as in early onset obesity, refers to an onset (e.g., first occurrence of one or more symptoms of a disorder, e.g., a disorder described herein, e.g., obesity) that occurs in a subject before adulthood, e.g., during childhood, e.g., when the subject is less 18 years of age or younger (e.g., 18, 17, 16, 15, 14, 13, 12, 11, 10, 9, 8, 7, 6, 5, 4, 3, 2, or 1 year of age or younger, or during adolescence, e.g., when the child is younger than 12 years of age or when the child is younger than 6 years of age).

As used herein, the term "metabolic syndrome" refers to a group of symptoms that occur together and increase the risk for coronary artery disease, stroke, and type 2 diabetes. According to the American Heart Association and the National Heart, Lung, and Blood Institute, metabolic syndrome also referred to as Syndrome X) is present if a subject has three or more of the following signs:
1) Blood pressure equal to or higher than 130/85 mmHg;
2) Fasting blood sugar (glucose) equal to or higher than 100 mg/dL;
3) Large waist circumference (length around the waist):
   - Men - 40 inches or more;
   - Women - 35 inches or more;
4) Low HDL cholesterol:
   - Men - under 40 mg/dL;
   - Women - under 50 mg/dL;
5) Triglycerides equal to or higher than 150 mg/dL.
Metabolic syndrome can be diagnosed by testing subject's blood pressure, blood glucose level, HDL cholesterol level, LDL cholesterol level, total cholesterol level, and triglyceride level.

As used herein, the term "agonist" refers to any chemical compound, either naturally occurring or synthetic, that, upon interacting with (*e.g*., binding to) its target, e.g., MC4R, raises the signaling activity of MC4R above its basal level. An agonist can be a superagonist (*i.e.* a compound that is capable of producing a greater maximal response than the endogenous agonist for the target receptor, and thus has an efficacy of more than 100%), a full agonist (*i.e.* a compound that elicits a maximal response following receptor occupation and activation) or a partial agonist (*i.e*. a compounds that can activate receptors but are unable to elicit the maximal response of the receptor system).

As used herein "treating" includes achieving one or more of the following results: reducing the body weight (as measured, for example, by a body mass index (BMI) and/or body weight), e.g., compared to a control (e.g., body weight before treatment or a predetermined body weight); reducing the waist circumference, e.g., compared to a control (e.g., waist circumference before treatment or a predetermined waist circumference); reducing the hunger level, e.g., compared to a control (e.g., hunger level before treatment or a predetermined hunger level); increasing the resting energy expenditure (REE), e.g., compared to a control (e.g., REE before treatment or a predetermined REE); decreasing the food intake, e.g., compared to a control level (e.g., before treatment or a predetermined food intake); ameliorating or improving a clinical symptom or indicators associated with a disorder described herein such as obesity, type-II diabetes, pre-diabetic condition, blood level of haemoglobin A1C (HblAc) above 6%, hyperinsulimenia, hyperlipidemia, insulin insensitivity, or glucose intolerance; delaying, inhibiting or preventing the progression of obesity and/or obesity related indications; or partially or totally delaying, inhibiting or preventing the onset or development of obesity or a obesity related indication. Delaying, inhibiting or preventing the progression of the obesity includes for example, delaying, inhibiting or preventing the progression of a subject having normal weight to obesity. In embodiments, a control is a value of a parameter measured before treatment by a MC4R agonist described herein or a predetermined value. The term "treating" further includes partially or totally reducing the risk for coronary artery disease, stroke, and type 2 diabetes associated with the metabolic syndrome as well as ameliorating or improving a clinical symptom or signs of metabolic syndrome associated with metabolic syndrome, such as any one or more of the five indicators listed above. For example, the term "treating" includes delaying, inhibiting or preventing the progression of parameters associated with the metabolic syndrome, including insulin resistance, glucose clearance and parameters of cardiovascular disease including heart rate and blood pressure.

As used herein "inhibition" or "inhibits" can include a reduction in a certain parameter, such as a parameter described herein. For example, inhibition of a parameter, e.g., activity, can be at least 5%, 10%, 20%, 30%, 40%, or more is included by this term. Thus, inhibition need not be 100%.

"Prophylactic treatment" refers to treatment before onset of obesity to prevent, inhibit or reduce its occurrence.

As used herein, the term "subject" refers to a mammal, e.g., a human. Subject can also refer to an animal in need of veterinary treatment, e.g., companion animals (e.g., dogs, cats, and the like), farm animals (e.g., cows, sheep, pigs, horses, and the like) and laboratory animals (e.g., rats, mice, guinea pigs, and the like).

As used herein, the term "mutation" can refer to an altered nucleic acid sequence of a gene or fragment thereof compared to a wild-type sequence. For example, a mutation can include a point mutation, frame-shift mutation, missense mutation, inversion, deletion, insertion, truncation, chromosomal translocation. In embodiments, a mutation can result in the gene or fragment thereof coding for a non-functional protein, a protein with reduced activity (or a partially functional protein), or a protein with altered activity. For example, a "loss of function" mutation refers to a mutation that results in the gene or fragment thereof coding for a non-functional protein, which has substantially reduced activity compared to its wild-type counterpart (e.g., a non-functional protein has less than 60%, 50% , 40% , 30% 20%, 10%, 9%, 8%, 7%, 6%, 5%, 4%, 3%, 2%, 1% or less activity than its wild-type counterpart). For example, "partial loss of function" mutation refers to a mutation that results in the gene or fragment thereof coding for a partially functional protein, which has reduced activity compared to its wild-type counterpart (e.g., a partially functional protein has less than 50% and greater than 10% of the activity of its wild-type counterpart).

As used herein "unit dosage" refers to a physically discrete unit suited as unitary doses for a subject to be treated. Each unit contains a predetermined quantity of active compound calculated to produce the desired therapeutic effect in association with the required pharmaceutical carrier.

As used herein "dosage" refers to a quantity or amount of a therapeutic agent. In some embodiments, a dosage is the amount administered to the subject in a single administration, e.g., in a single injection, a single infusion, or single administration of one or more unit dosages. In embodiments, a dosage is the amount administered to the subject in multiple administrations, e.g., multiple injections, multiple infusions, or multiple administrations of one or more unit dosages. In other embodiments, a dosage can refer to the total amount administered to the subject in a certain time period, e.g., per day. In such examples, the dosage is typically referred to as "daily dosage" or dosage in terms of quantity per day.

As used herein "hunger" or "hunger level" refers to a subject's appetite, desire to consume food, or perceived need for food. In embodiments, the hunger or hunger level of a subject can be quantified by using a scale to obtain a hunger score. In embodiments, the scale for hunger assigns a higher score for a subject that more frequently (e.g., often or always) feels unbearable hunger and a lower score for a subject that less frequently (e.g., sometimes or never) feels unbearable hunger. See, e.g., Sibilia. Psychologicol Topics 19 (2010), 2, 341-354. For example, a Likert scale for hunger can be used that assigns scores from 0 to 10 points (0=no hunger; 10=severe hunger). In other examples, a Likert scale for hunger can be used that assigns scores from 1 to 4 points, where a subject who never feels unbearable hunger is assigned a score of 1, where a subject who sometimes feels unbearable hunger is assigned a score of 2, where a subject who often feels unbearable hunger is assigned a score of 3, and where a subject who always feels unbearable hunger is assigned a score of 4. See i.d.

### Disorders

In accordance with the methods and compositions described herein, in some embodiments, a MC4R agonist, e.g., MC4R agonist described herein is used to treat a disorder, such as a metabolic disorder, e.g., obesity, hyperphagia, or metabolic syndrome.

### Prader Willi Syndrome (PWS)

Prader Willi Syndrome (PWS) is a rare genetic disease with a prevalence ranging from approximately one in 8,000 to one in 25,000 patients in the U.S. A hallmark of PWS is severe hyperphagia-an overriding physiological drive to eat-leading to severe obesity and other complications. Obesity is one of the greatest health threats to PWS patients, and hyperphagia impairs the ability of PWS patients to live independently, requiring costly and constant supervision to prevent overeating. Without supervision, these patients are likely to die prematurely as a result of choking, stomach rupture, or from complications caused by morbid obesity. Currently, there are no approved treatments for the obesity and hyperphagia associated with PWS.

Symptoms of PWS include infantile hypotonia with failure to thrive, rapid weight gain and overeating during childhood, as well as intellectual disability, developmental delay, short stature, hypogonadism. Diagnostic criteria for PWS are described, e.g., in Holm et al. Pediatrics 91(1993):398-402.

### Outcomes

In embodiments, methods described herein result in one or more outcomes, including a reduction of weight (e.g., body weight), a reduction in hunger level, no detectable decrease in energy expenditure (e.g., resting energy expenditure), an increase in energy expenditure (e.g., resting energy expenditure), a reduction in daily/weekly/monthly food intake, a reduction in waist circumference, no detectable increase in blood pressure, or a reduction in blood pressure in a subject, e.g., relative to a control.

In embodiments, the control is the measurement of the parameter in the subject prior to administration of (treatment with) a MC4R agonist. In embodiments, the control is a predetermined value, e.g., the value of the parameter in an average obese human population, e.g., of like age and gender as the subject; or the value of the parameter measured in the subject at a previous time point (e.g., at a previous visit, e.g., to a physician, medical facility or laboratory).

In embodiments, the outcome (e.g., the reduction, increase, no detectable decrease, or no detectable increase in a given parameter) is measured in the subject 1, 2, 3, 4, 5, 6, 7, 8, 9, 10 weeks or more after initation of treatment with a MC4R agonist. In other embodiments, the outcome (e.g., the reduction, increase, no detectable decrease, or no detectable increase in a given parameter) is measured in the subject over a period of time (e.g., over a period of 1-2 weeks, 2-4 weeks, 4-6 weeks, 6-8 weeks, 8-12 weeks, or 12-16 weeks or longer) during a course of treatment.

In embodiments, methods described herein result in a reduction of weight (e.g., body weight) in the subject compared to a control (e.g., weight of the subject before treatment or a predetermined value, e.g., average weight of an obese human population of like age and gender as the subject not subjected to therapeutic intervention, or the weight of the subject at a previous measurement, e.g., at a previous visit). In embodiments, the reduction is about 0.5 to 1 kg to 0.5 to 3 kg after 1 week of treatment, about 1 kg to 6 kg after 2 weeks of treatment, about 2 kg to 12 kg after 4 weeks of treatment, about 4 kg to 24 kg after 8 weeks of treatment, or about 8 kg to 48 kg after 16 weeks of treatment. In embodiments, the reduction is at a rate of loss of about .5 to 2 kg/week, e.g., about 2 kg/week, e.g., over a period of 1-2 weeks of treatment or longer, 2-4 weeks of treatment or longer, 4-8 weeks of treatment or longer, 8-16 weeks of treatment, or 16-32 weeks of treatment, or longer.

Measurement of weight, e.g., body weight, can be performed using standard methods in the art.

In embodiments, methods described herein result in a reduction in hunger level in the subject compared to a control (e.g., hunger level of the subject before treatment or a predetermined hunger level, e.g., average hunger level of an obese human population of like age and gender as the subject or the hunger level of the subject at a previous measurement, e.g., at a previous visit). In embodiments, the methods described herein result in abolishment of hunger in the subject.

In embodiments, hunger is measured by a scale, such as a Likert hunger scale, which ranges from 0 to 10 and is described herein. In embodiments, methods described herein result in a reduction in hunger score in the subject compared to a control (e.g., hunger level of the subject before treatment or a predetermined hunger level, e.g., average hunger level of an obese human population of like age and gender as the subject or the hunger level of the subject at a previous measurement, e.g., at a previous visit). In embodiments, methods described herein result in a lower score on the Likert hunger scale, e.g., a lower score by at least 1, 2, 3, 4, 5, 6, 7, 8, 9 or 10 points, compared to the control (e.g., hunger level of the subject before treatment or a predetermined hunger level, e.g., average hunger level of an obese human population of like age and gender as the subject or the hunger level of the subject at a previous measurement, e.g., at a previous visit). In embodiments, methods described herein result in a score of 0 on the Likert hunger scale after treatment.

In embodiments, the reduction in hunger level is measured/observed after 1 to 2 weeks of treatment or longer, 2-4 weeks of treatment or longer, 4-8 weeks of treatment or longer, or 8-16 weeks of treatment or longer.

REE is a measure of the basal metabolic rate of the subject and can be determined using methods such as those described in Chen et al. J. Clin. Endocrinol. Metab. 100.4(2015):1639-45. In embodiments, the REE can be determined by placing the subject in a whole-room indirect calorimeter (also called a metabolic chamber) at a certain time after treatment (e.g., after 3, 4, 5, 6, 7 days, or 1, 2, 3, 4, or more weeks). In embodiments, the REE is measured in 30-minute measurements periods, and in some cases, REE values from several 30-minute periods are averaged to generate an average REE. In embodiments, the REE can be determined after a 10-12 hour fasting period, at thermoneutrality (e.g., around 25 deg C), where the subject is awake without psychological or physical stress. In embodiments, REE is measured in units of energy per unit time (e.g., kcal/h or kcal/day). In embodiments, the REE is measured relative to kg lean body mass in a subject (e.g., REE/kg lean mass), e.g., as described in the Examples.

In embodiments, methods described herein result in no change or no decrease in energy expenditure, e.g., resting energy expenditure (REE), in the subject over an hourly, daily (e.g., in 24 hours), weekly (e.g., in 7 days), or monthly (e.g., in 30 days) period compared to a control REE (e.g., the REE in the subject prior to treatment or a predetermined REE, e.g., average REE of an obese human population of like age and gender and normalized for weight as the subject or the REE of the subject at a previous measurement, e.g., previous visit), e.g., as measured after 3, 4, 5, 6, 7 days, or 1, 2, 3, 4, or more weeks of treatment.

In embodiments, methods described herein result in no detectable change or no detectable decrease in energy expenditure, e.g., resting energy expenditure (REE) per kg lean body mass, in the subject over an hourly, daily (e.g., in 24 hours), weekly (e.g., in 7 days), or monthly (e.g., in 30 days) period compared to the control REE (e.g., the REE in the subject prior to treatment or a predetermined REE, e.g., average REE of an obese human population of like age and gender as the subject or the REE of the subject at a previous measurement, e.g., previous visit), e.g., as measured after 3, 4, 5, 6, 7 days, or 1, 2, 3, 4, or more weeks of treatment.

In embodiments, methods described herein result in an increase in energy expenditure, e.g., resting energy expenditure (REE), in the subject over a hourly, daily (e.g., in 24 hours), weekly (e.g., in 7 days), or monthly (e.g., in 30 days) period compared to a control REE (e.g., the REE in the subject prior to treatment or a predetermined REE, e.g., average REE of an obese human population of like age and gender and normalized for weight as the subject or the REE of the subject at a previous measurement, e.g., previous visit), e.g., as measured after 3, 4, 5, 6, 7 days, or 1, 2, 3, 4, or more weeks of treatment.

In embodiments, the increase in REE in the subject is at least 20 kcal/day (e.g., at least 20, 30, 40, 50, 60, 70, 80, 90, 100, 110, 120, 130, 140, 150 kcal/day or more), e.g., as measured after 3, 4, 5, 6, 7 days, or 1, 2, 3, 4, or more weeks of treatment.

In embodiments, the increase in REE in the subject is at least 2% (e.g., at least 2%, 3%, 4%, 5%, 6%, 7%, 8%, 9%, 10%, 11%, 12%, 13%, 14%, 15% or more), e.g., as measured after 3, 4, 5, 6, 7 days, or 1, 2, 3, 4, or more weeks of treatment, compared to the REE in the subject prior to treatment.

In embodiments, the REE in the subject (e.g., adult subject) after treatment with a MC4R agonist (e.g., after 3, 4, 5, 6, 7 days, or 1, 2, 3, 4, or more weeks of treatment) is at least 1800 kcal/day (e.g., at least 1800, 1825, 1850, 1875, 1900, 1925, 1950, 1975, 2000, 2025, 2050, 2100, 2150, 2200, 2250, 2300, 2400 kcal/day, or more), e.g., for an adult subject. In embodiments, the REE in the subject (e.g., pediatric subject) after treatment with a MC4R agonist (e.g., after 3, 4, 5, 6, 7 days, or 1, 2, 3, 4, or more weeks of treatment) is at least 200 kcal/day (e.g., at least 200, 225, 250, 275, 300, 325, 350, 375, 400, 450, 500 kcal/day or more), e.g., for pediatric patients.

In embodiments, methods described herein result in a reduction in food intake by the subject compared to a control (e.g., the food intake of the subject prior to treatment or a predetermined food intake level, e.g., the food intake of an average human obese population or the food intake of the subject at a previous measurement, e.g., at a previous visit), e.g., where the food intake is measured as daily food intake or food intake over a period of 24 hours, or one week. In embodiments, the reduction is at least 100 kilocalories, e.g., at least 100, 125, 150, 175, 200, 225, 250, 275, 300, 325, 350, 375, 400, 425, 450, 475, 500, 525, 550, 575, 600, 1000 kilocalories or more, e.g., for daily food intake or food intake over a period of 24 hours, or one week, or 30 days or for longer time periods, e.g., for an adult subject. In embodiments, mean food intake can decrease from a baseline at or above about 100 kcal/kg/day to about 90, 80, 70, 60, 50, 40, 30, 20 or 10 kcal/kg/day or lower after treatment with a MC4R agonist, e.g., in a pediatric subject at about 1 year of age. In embodiments, mean food intake can decrease from a baseline at or above about 40 kcal/kg/day to about 35, 30, 20 or 10 kcal/kg/day or lower after treatment with a MC4R agonist, e.g., in a pediatric subject in late adolescence.

Food intake can be determined by standard methods, e.g., as described in Rutishauser. Pub. Health Nutr. 8.7A(2005):1100-07.

In embodiments, methods described herein result in a reduction in waist circumference of the subject compared to a control (e.g., the waist circumference of the subject prior to treatment or the waist circumference of the subject at a previous measurement, e.g., previous visit), as measured 1, 2, 3, 4, 5, 6, 7, 8, 9, 10 weeks or more after initiation of treatment.

In embodiments, the reduction in waist circumference is at least 2 cm (e.g., at least 2, 3, 4, 5, 6, 7, 8, 9, 10 cm or more) in the subject (e.g., adult subject) compared to a control (e.g., the waist circumference of the subject prior to treatment or a predetermined waist circumference, e.g., the waist circumference of an average obese human population of like age and gender or the waist circumference of the subject at a previous measurement, e.g., previous visit), as measured 1, 2, 3, 4, 5, 6, 7, 8, 9, 10 weeks or more after initiation of treatment.

In embodiments, the waist circumference is measured using standard methods. In embodiments, the waist circumference is the largest circumference around a subject's midsection, e.g., around a subject's abdomen. In other embodiments, the waist circumference is measured around the natural waist (e.g., in between the lowest rib and the top of the hip bone), the umbilicus, or at the narrowest point of the midsection.

In embodiments, methods described herein result in no detectable increase in blood pressure (e.g., diastolic and/or systolic blood pressure) of the subject compared to a control blood pressure (e.g., the blood pressure of the subject prior to treatment or a predetermined blood pressure, e.g., the blood pressure of an average obese human population of like age and gender or the blood pressure of the subject at a previous measurement, e.g., previous visit), as measured 1, 2, 3, 4, 5, 6, 7, 8, 9, 10 weeks or more after initiation of treatment.

In embodiments, methods described herein result in a reduction in blood pressure (e.g., diastolic and/or systolic blood pressure) of the subject a control blood pressure (e.g., the blood pressure of the subject prior to treatment or a predetermined blood pressure, e.g., the blood pressure of an average obese human population of like age and gender or the blood pressure of the subject at a previous measurement, e.g., previous visit), as measured 1, 2, 3, 4, 5, 6, 7, 8, 9, 10 weeks or more after initiation of treatment.

In embodiments, the reduction in blood pressure, e.g., systolic blood pressure, is at least 1, 2 or 3 mmHg (e.g., at least 3, 3.5, 4, 4.5, 5, 5.5, 6, 6.5, 7 mmHg or more) compared to the blood pressure of the subject prior to treatment, as measured 1, 2, 3, 4, 5, 6, 7, 8, 9, 10 weeks or more after initiation of treatment.

In embodiments, the reduction in blood pressure, e.g., diastolic blood pressure, is at least 4 mmHg (e.g., at least 4, 7, 7.5, 8, 8.5, 9, 9.5, 10 mmHg or more) compared to the blood pressure of the subject prior to treatment, as measured 1, 2, 3, 4, 5, 6, 7, 8, 9, 10 weeks or more after initiation of treatment.

In embodiments, the methods described herein do not result in an adverse effect on heart rate or blood pressure.

### Subjects

In accordance with any method described herein, in certain embodiments, the subject is obese, e.g., prior to administration of an agonist described herein, e.g., at the time the agonist is prescribed, or at the time of the first administration of the agonist. In embodiments, the subject is a severely obese, pediatric or adult patient e.g., prior to administration of an agonist described herein, e.g., at the time the agonist is prescribed or at the time of the first administration of the agonist. In embodiments, the subject is hyperphagic, e.g., prior to administration of an agonist described herein, e.g., at the time the agonist is prescribed, or at the time of the first administration of the agonist.

In embodiments, the subject (e.g., adult subject) has a body mass index (BMI) greater than 25 kg/m² or 30 kg/m² (e.g., ≥ 25, 26, 27, 28, 29, 30, 31, 32, 33, 34, 35, 36, 37, 38, 39, 40, 41, 42, 43, 44, 45, 46, 47, 48, 49, 50 kg/m² or greater) prior to administration of the agonist, e.g., at the time the agonist is prescribed, or at the time of the first administration.

In embodiments, the subject (e.g., pediatric subject) has a body mass index (BMI) higher than 85-95 percentile prior to administration of the agonist, e.g., at the time the agonist is prescribed, or at the time of the first administration.

In embodiments, the subject has a body weight of at least about 5 kg, e.g., at least about 5 kg, 10 kg, 20kg, 30, 40, 50, 60, 70, 80, 90, 100, 110, 120, 130, 140,145, 150, 155, 160, 165, 170, 175, 180, 185, 190, 200, 205, 210, 215, 220 kg or greater, e.g., prior to administration of the agonist, e.g., at the time the agonist is prescribed, or at the time of the first administration. In embodiments, the subject has a body weight of a least 20 kg, at least 60 kg, or at least 100 kg, e.g., prior to administration of the agonist, e.g., at the time the agonist is prescribed, or at the time of the first administration.

In embodiments, the subject is an adult, e.g., 18 years of age or older, e.g., 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, or older.

In embodiments, the subject is a pediatric subject, e.g., less 18 years of age or younger (e.g., 18, 17, 16, 15, 14, 13, 12, 11, 10, 9, 8, 7, 6, 5, 4, 3, 2, or 1 year of age or younger.

In yet another aspect, the present invention provides a method of eliciting an agonist effect from a melanocortin receptor in a subject in need thereof. The method comprises administering to the subject an effective amount of a compound of formula (I) or formula (II) as defined hereinabove, or pharmaceutically acceptable salts thereof.

In another aspect, the present invention provides a method of eliciting an agonist effect from a melanocortin receptor in a subject in need thereof. The method comprises administering to the subject an effective amount of a compound of formula (I) or formula (II) as defined hereinabove, or pharmaceutically acceptable salts thereof, wherein the compound is a selective melanocortin 4 receptor agonist.

In a further aspect, the present invention provides a method of eliciting an agonist or an antagonist effect from a melanocortin receptor in a subject in need thereof. The method comprises administering to the subject an effective amount of a compound of formula (I) or formula (II) as defined hereinabove, or pharmaceutically acceptable salts thereof, wherein the compound is a selective melanocortin 4 receptor agonist.

In yet another aspect, the present invention provides a method of eliciting an agonist effect from a melanocortin receptor in a subject in need thereof. The method comprises administering to the subject an effective amount of a compound of formula (I) or formula (II) as defined hereinabove, or pharmaceutically acceptable salts thereof, wherein the compound is a selective melanocortin 4 receptor agonist.

In another aspect, the present invention provides a method of treating an acute or chronic inflammatory disease or medical condition such as general inflammation, inflammatory bowel disease, brain inflammation, sepsis and septic shock by eliciting an agonist effect from a melanocortin receptor by administering an effective amount of a compound of formula (I) or formula (II) as defined hereinabove, or pharmaceutically acceptable salts thereof.

In another aspect, the present invention provides a method of treating a disease or medical condition with an autoimmune component such as rheumatoid arthritis, gouty arthritis and multiple sclerosis by eliciting an agonist effect from a melanocortin receptor by administering an effective amount of a compound of formula (I) or formula (II) as defined hereinabove, or pharmaceutically acceptable salts thereof.

In another aspect, the present invention provides a method of treating a metabolic disease or medical condition accompanied by weight gain such as obesity, feeding disorders and Prader-Willi Syndrome by eliciting an agonist effect from a melanocortin receptor by administering an effective amount of a compound of formula (I) or formula (II) as defined hereinabove, or pharmaceutically acceptable salts thereof. In a further aspect of the foregoing method, the disease or condition treated is obesity. In yet a further aspect of the foregoing method, the disease or condition treated is a feeding disorder characterized by hyperphagiay and/or obesity.

In another aspect, the present invention provides a method of decreasing food intake, decreasing body weight or a combination thereof, by eliciting an agonist effect from a melanocortin receptor by administering an effective amount of a compound of formula (I) or formula (II) as defined hereinabove, or pharmaceutically acceptable salts thereof. In an embodiment, the present invention provides a method of decreasing food intake, decreasing body weight or a combination thereof, by eliciting an agonist effect from a melanocortin receptor by administering an effective amount of a compound of formula (I) or formula (II), or pharmaceutically acceptable salts thereof.

In another embodiment, the present invention provides a method of decreasing food intake, decreasing body weight or a combination thereof, by eliciting an agonist effect from a melanocortin receptor by administering an effective amount of a compound of formula (I) or (II), or pharmaceutically acceptable salts thereof.

In another embodiment, the present invention provides a method of decreasing food intake, decreasing body weight or a combination thereof, by eliciting an agonist effect from a melanocortin receptor by administering an effective amount of a compound of formula (I) or (II), or pharmaceutically acceptable salts thereof. In another embodiment, the present invention provides a method of decreasing food intake, decreasing body weight or a combination thereof, by eliciting an agonist effect from a melanocortin receptor by administering an effective amount of a compound of formula (I) or formula II, or pharmaceutically acceptable salts thereof.

In another aspect, the present invention provides a method of decreasing appetite without compromising body weight by administering an effective amount of a compound of formula (I) or formula (II) as defined hereinabove, or pharmaceutically acceptable salts thereof. In another aspect, the present invention provides a method of decreasing food consumption while decreasing body weight by administering an effective amount of a compound of formula (I) or formula (II) as defined hereinabove, or pharmaceutically acceptable salts thereof.

In another aspect, the present invention provides a method of treating a reproductive or sexual medical condition such as endometriosis, uterine bleeding, sexual dysfunction, erectile dysfunction and decreased sexual response in females by eliciting an agonist effect from a melanocortin receptor by administering an effective amount of a compound of formula (I) or formula (II) as defined hereinabove, or pharmaceutically acceptable salts thereof.

In another aspect, the present invention provides a method of treating a disease or medical condition resulting from treatment or insult to an organism such as organ transplant rejection, ischemia and reperfusion injury, wounding and spinal cord injury, and weight loss due to a medical procedure selected from the group consisting of chemotherapy, radiation therapy, temporary or permanent immobilization and dialysis by eliciting an agonist effect from a melanocortin receptor by administering an effective amount of a compound of formula (I) or formula (II) as defined hereinabove, or pharmaceutically acceptable salts thereof.

In another aspect, the present invention provides a method of treating a cardiovascular disease or medical condition such as hemorrhagic shock, cardiogenic shock, hypovolemic shock, cardiovascular disorders and cardiac cachexia by eliciting an agonist effect from a melanocortin receptor by administering an effective amount of a compound of formula (I) or formula (II) as defined hereinabove, or pharmaceutically acceptable salts thereof.

In another aspect, the present invention provides a method of treating a pulmonary disease or medical condition such as acute respiratory distress syndrome, pulmonary fibrosis, chronic obstructive pulmonary disease and asthma by eliciting an agonist effect from a melanocortin receptor by administering an effective amount of a compound of formula (I) or formula (II) as defined hereinabove, or pharmaceutically acceptable salts thereof.

In another aspect, the present invention provides a method of enhancing immune tolerance or treating allergies by eliciting an agonist effect from a melanocortin receptor by administering an effective amount of a compound of formula (I) or formula (II) as defined hereinabove, or pharmaceutically acceptable salts thereof.

In another aspect, the present invention provides a method of treating dermatological disease or medical condition such as psoriasis, erythropoietic protoporphyria, skin pigmentation depletion, acne and keloid formation by eliciting an agonist effect from a melanocortin receptor by administering an effective amount of a compound of formula (I) or formula (II) as defined hereinabove, or pharmaceutically acceptable salts thereof.

In another aspect, the present invention provides a method of treating a behavioral or central nervous system or neuronal disease or medical condition such as anxiety, depression, memory dysfunction and neuropathic pain by eliciting an agonist effect from a melanocortin receptor by administering an effective amount of a compound of formula (I) or formula (II) as defined hereinabove, or pharmaceutically acceptable salts thereof.

In another aspect, the present invention provides a method of treating a renal disease or medical condition such as renal cachexia and natriuresis by eliciting an agonist effect from a melanocortin receptor by administering an effective amount of a compound of formula (I) or formula (II) as defined hereinabove, or pharmaceutically acceptable salts thereof.

In another aspect, the present invention provides a method of modulating a normalizing or homeostatic activity such as ovarian weight, placental development, prolactin secretion, FSH secretion, intrauterine fetal growth, parturition, spermatogenesis, thyroxin release, aldosterone synthesis and release, gonadal development, body temperature, blood pressure, heart rate, vascular tone, brain blood flow, blood glucose levels, sebum secretion, pheromone secretion, motivation, learning and behavior, pain perception, neuroprotection and nerve growth by eliciting an agonist effect from a melanocortin receptor by administering an effective amount of a compound of formula (I) or formula (II) as defined hereinabove, or pharmaceutically acceptable salts thereof.

In another aspect, the present invention provides a method of modulating a normalizing or homeostatic activity such as bone metabolism, bone formation and bone development by eliciting an agonist effect from a melanocortin receptor by administering an effective amount of a compound of formula (I) or formula (II) as defined hereinabove, or pharmaceutically acceptable salts thereof.

In another aspect, the present invention provides a method of inhibiting alcohol consumption, for reducing alcohol consumption, for treating alcoholism, or for treating alcohol abuse by eliciting an agonist effect from a melanocortin receptor by administering an effective amount of a compound of formula (I) or formula (II) as defined hereinabove, or pharmaceutically acceptable salts thereof. In a further aspect of the foregoing method, the compound is a selective melanocortin 4 receptor agonist.

In a further aspect, the present invention provides the use of a therapeutically effective amount of a melanocortin 4 receptor agonist compound according formula (I) or formula (II) as defined hereinabove, or pharmaceutically acceptable salts thereof, for the manufacture of a medicament useful to treat a disease and/or medical condition selected from the group consisting of acute and chronic inflammatory diseases such as general inflammation, inflammatory bowel disease, brain inflammation, sepsis and septic shock; diseases with an autoimmune component such as rheumatoid arthritis, gouty arthritis and multiple sclerosis; metabolic diseases and medical disorders accompanied by weight gain such as obesity, feeding disorders and Prader-Willi Syndrome; metabolic diseases and medical disorders accompanied by weight loss such as anorexia, bulimia, AIDS wasting, cachexia, cancer cachexia and wasting in frail elderly; diabetes, diabetalogical related conditions and complications of diabetes such as retinopathy; neoplastic proliferation such as skin cancer and prostate cancer; reproductive or sexual medical conditions such as endometriosis and uterine bleeding in women, sexual dysfunction, erectile dysfunction and decreased sexual response in females; diseases or conditions resulting from treatment or insult to the organism such as organ transplant rejection, ischemia and reperfusion injury, spinal cord injury and wounding, as well as weight loss caused chemotherapy, radiation therapy, temporary or permanent immobilization or dialysis; cardiovascular diseases or conditions such as hemorrhagic shock, cardiogenic shock, hypovolemic shock, cardiovascular disorders and cardiac cachexia; pulmonary diseases or conditions such as acute respiratory distress syndrome, chronic obstructive pulmonary disease, asthma and pulmonary fibrosis; to enhance immune tolerance and to combat assaults to the immune system such as those associated with certain allergies or organ transplant rejection; treatment of dermatological diseases and conditions such as psoriasis, skin pigmentation depletion, acne, keloid formation and skin cancer; behavioral, central nervous system and neuronal disorders such as anxiety, depression, memory dysfunction, and neuropathic pain; and renal conditions or diseases such as the treatment of renal cachexia and natriuresis.

In a further aspect, the present invention provides the use of a therapeutically effective amount of a melanocortin 4 receptor agonist compound according formula (I) or formula (II) as defined hereinabove, or pharmaceutically acceptable salts thereof, for the manufacture of a medicament useful to modulate normalizing or homeostatic activities such as ovarian weight, placental development, prolactin secretion, FSH secretion, intrauterine fetal growth, parturition, spermatogenesis, gonadal development, thyroxin release, aldosterone synthesis and release, body temperature, blood pressure, heart rate, vascular tone, brain blood flow, blood glucose levels, sebum secretion, pheromone secretion, motivation, learning and behavior, pain perception, neuroprotection, nerve growth, bone metabolism, bone formation and bone development.

It will be appreciated that therapeutic interventions addressing both normal physiological and pathophysiological processes which utilize the melanocortin receptors are also contemplated.

The compounds of formulas (I) or (II) are ligands for at least one of the melanocortin receptors (MC1-R, MC2-R, MC3-R, MC4-R and MC5-R) and a selection thereof were tested for their ability to act as a ligand in the in vitro assay described below.

In some aspects, provided herein is also a method of evaluating a subject, e.g., for likely responsiveness to a MC4R agonist described herein. In some embodiments, the method comprises acquiring information about the genotype of the subject.

In embodiments, the identification of the subject having a defect, e.g., genetic defect, e.g., mutation, indicates that the subject is likely to respond (e.g., with an improvement in one or more symptoms) to a MC4R agonist, e.g., a MC4R agonist described here. In embodiments, an improvement in a symptom can include an outcome described herein. For example, an improvement in a symptom can include a reduction of weight (e.g., body weight), a reduction in hunger level, no detectable decrease in energy expenditure (e.g., resting energy expenditure), an increase in energy expenditure (e.g., resting energy expenditure), a reduction in daily/weekly/monthly food intake, or a reduction in waist circumference, e.g., relative to a control.

In embodiments, methods described herein further comprise providing a report that identifies the presence or absence of the genetic defect and in some cases an identifier for the subject. In embodiments, the report provides a recommendation on potential therapeutic options, likely effectiveness of a therapeutic option, and/or recommendations/instructions for administration of the therapeutic option (e.g., a MC4R agonist described herein).

### MC4R agonists

The invention provides MC4R agonists according to Formula I: 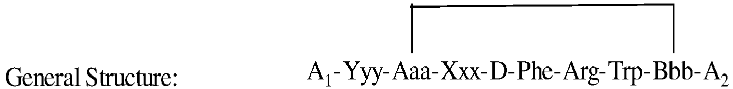
Aaa, Bbb = Selected from Cys, hCys, Pen; capable of establishing a disulfide bridge or
   Glu, Asp, Lys, Orn, Dpr, Dbu capable of establishing a lactam bridge
Xxx = Asn, Gln, Ser, Thr
Yyy = Lys, Arg, D-Lys, D-Arg
A₁ = H, Ac
A₂ = OH, NH₂

Ac is acyl, e.g., acetyl.

In embodiments, an MC4R agonist has a structure according to Formula II: 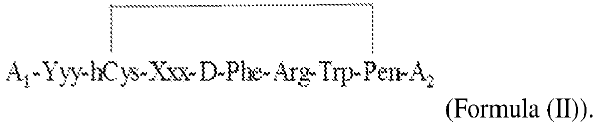
where Xxx is Asn, Gln, Ser, or Thr,
where A₁ is H or Ac,
where A₂ is OH or NH₂, and
where Yyy is Lys, Arg, D-Lys, or D-Arg.

In embodiments, the MC4R agonist is chosen from one or more of the following compounds, (or pharmaceutically acceptable salt thereof):
001554C Ac-Arg-(hCys-Asn-D-Phe-Arg-Trp-Pen)-NH₂ (SEQ ID NO: 1)
001555C Ac-Arg-(hCys-Gln-D-Phe-Arg-Trp-Pen)-NH₂ (SEQ ID NO: 2)
001556C Ac-Arg-(hCys-Ser-D-Phe-Arg-Trp-Pen)-NH₂ (SEQ ID NO: 3)
001574C: Ac-Arg-(hCys-Thr-D-Phe-Arg-Trp-Pen)-NH₂ (SEQ ID NO: 4)
001576C: Ac-Arg-(Glu-Gln-D-Phe-Arg-Trp-Apr)-NH₂ (SEQ ID NO: 5)
Ac-Arg-(hCys-Asn-D-Phe-Arg-Trp-Pen)-OH (SEQ ID NO: 6)
H-Arg-(hCys-Asn-D-Phe-Arg-Trp-Pen)-NH₂ (SEQ ID NO: 7)
H-Arg-(hCys-Asn-D-Phe-Arg-Trp-Pen)-OH (SEQ ID NO: 8)
Ac-D-Arg-(hCys-Asn-D-Phe-Arg-Trp-Pen)-NH₂ (SEQ ID NO: 9)
H-D-Arg-(hCys-Asn-D-Phe-Arg-Trp-Pen)-NH₂ (SEQ ID NO: 10)
Ac-D-Arg-(hCys-Asn-D-Phe-Arg-Trp-Pen)-OH (SEQ ID NO:11)
H-D-Arg-(hCys-Asn-D-Phe-Arg-Trp-Pen)-OH (SEQ ID NO: 12)
Ac-Lys-(hCys-Asn-D-Phe-Arg-Trp-Pen)-NH₂ (SEQ ID NO: 13)
Ac-Lys-(hCys-Asn-D-Phe-Arg-Trp-Pen)-OH (SEQ ID NO: 14)
H-Lys-(hCys-Asn-D-Phe-Arg-Trp-Pen)-NH₂ (SEQ ID NO:15)
H-Lys-(hCys-Asn-D-Phe-Arg-Trp-Pen)-OH (SEQ ID NO: 16)
Ac-D-Lys-(hCys-Asn-D-Phe-Arg-Trp-Pen)-NH₂ (SEQ ID NO: 17)
H-D-Lys-(hCys-Asn-D-Phe-Arg-Trp-Pen)-NH₂ (SEQ ID NO: 18)
Ac-D-Lys-(hCys-Asn-D-Phe-Arg-Trp-Pen)-OH (SEQ ID NO: 19)
H-D-Lys-(hCys-Asn-D-Phe-Arg-Trp-Pen)-OH (SEQ ID NO: 20)
Ac-Arg-(hCys-Gln-D-Phe-Arg-Trp-Pen)-OH (SEQ ID NO: 21)
H-Arg-(hCys-Gln-D-Phe-Arg-Trp-Pen)-NH₂ (SEQ ID NO: 22)
H-Arg-(hCys-Gln-D-Phe-Arg-Trp-Pen)-OH (SEQ ID NO: 23)
Ac-D-Arg-(hCys-Gln-D-Phe-Arg-Trp-Pen)-NH₂ (SEQ ID NO: 24)
H-D-Arg-(hCys-Gln-D-Phe-Arg-Trp-Pen)-NH₂ (SEQ ID NO: 25)
Ac-D-Arg-(hCys-Gln-D-Phe-Arg-Trp-Pen)-OH (SEQ ID NO: 26)
H-D-Arg-(hCys-Gln-D-Phe-Arg-Trp-Pen)-OH (SEQ ID NO: 27)
Ac-Lys-(hCys-Gln-D-Phe-Arg-Trp-Pen)-NH₂ (SEQ ID NO: 28)
Ac-Lys-(hCys-Gln-D-Phe-Arg-Trp-Pen)-OH (SEQ ID NO: 29)
H-Lys-(hCys-Gln-D-Phe-Arg-Trp-Pen)-NH₂ (SEQ ID NO: 30)
H-Lys-(hCys-Gln-D-Phe-Arg-Trp-Pen)-OH (SEQ ID NO: 31)
Ac-D-Lys-(hCys-Gln-D-Phe-Arg-Trp-Pen)-NH₂ (SEQ ID NO: 32)
H-D-Lys-(hCys-Gln-D-Phe-Arg-Trp-Pen)-NH₂ (SEQ ID NO: 33)
Ac-D-Lys-(hCys-Gln-D-Phe-Arg-Trp-Pen)-OH (SEQ ID NO: 34)
H-D-Lys-(hCys-Gln-D-Phe-Arg-Trp-Pen)-OH (SEQ ID NO: 35)
Ac-Arg-(hCys-Ser-D-Phe-Arg-Trp-Pen)-OH (SEQ ID NO: 36)
H-Arg-(hCys-Ser-D-Phe-Arg-Trp-Pen)-NH₂ (SEQ ID NO: 37)
H-Arg-(hCys-Ser-D-Phe-Arg-Trp-Pen)-OH (SEQ ID NO: 38)
Ac-D-Arg-(hCys-Ser-D-Phe-Arg-Trp-Pen)-NH₂ (SEQ ID NO: 39)
H-D-Arg-(hCys-Ser-D-Phe-Arg-Trp-Pen)-NH₂ (SEQ ID NO: 40)
Ac-D-Arg-(hCys-Ser-D-Phe-Arg-Trp-Pen)-OH (SEQ ID NO:41)
H-D-Arg-(hCys-Ser-D-Phe-Arg-Trp-Pen)-OH (SEQ ID NO:42)
Ac-Lys-(hCys-Ser-D-Phe-Arg-Trp-Pen)-NH₂ (SEQ ID NO: 43)
Ac-Lys-(hCys-Ser-D-Phe-Arg-Trp-Pen)-OH (SEQ ID NO:44)
H-Lys-(hCys-Ser-D-Phe-Arg-Trp-Pen)-NH₂ (SEQ ID NO: 45)
H-Lys-(hCys-Ser-D-Phe-Arg-Trp-Pen)-OH (SEQ ID NO: 46)
Ac-D-Lys-(hCys-Ser-D-Phe-Arg-Trp-Pen)-NH₂ (SEQ ID NO: 47)
H-D-Lys-(hCys-Ser-D-Phe-Arg-Trp-Pen)-NH₂ (SEQ ID NO: 48)
Ac-D-Lys-(hCys-Ser-D-Phe-Arg-Trp-Pen)-OH (SEQ ID NO: 49)
H-D-Lys-(hCys-Ser-D-Phe-Arg-Trp-Pen)-OH (SEQ ID NO: 50)
Ac-Arg-(hCys-Thr-D-Phe-Arg-Trp-Pen)-OH (SEQ ID NO: 51)
H-Arg-(hCys-Thr-D-Phe-Arg-Trp-Pen)-NH₂ (SEQ ID NO: 52)
H-Arg-(hCys-Thr-D-Phe-Arg-Trp-Pen)-OH (SEQ ID NO: 53)
Ac-D-Arg-(hCys-Thr-D-Phe-Arg-Trp-Pen)-NH₂ (SEQ ID NO: 54)
H-D-Arg-(hCys-Thr-D-Phe-Arg-Trp-Pen)-NH₂ (SEQ ID NO: 55)
Ac-D-Arg-(hCys-Thr-D-Phe-Arg-Trp-Pen)-OH (SEQ ID NO: 56)
H-D-Arg-(hCys-Thr-D-Phe-Arg-Trp-Pen)-OH (SEQ ID NO: 57)
Ac-Lys-(hCys-Thr-D-Phe-Arg-Trp-Pen)-NH₂ (SEQ ID NO: 58)
Ac-Lys-(hCys-Thr-D-Phe-Arg-Trp-Pen)-OH (SEQ ID NO: 59)
H-Lys-(hCys-Thr-D-Phe-Arg-Trp-Pen)-NH₂ (SEQ ID NO: 60)
H-Lys-(hCys-Thr-D-Phe-Arg-Trp-Pen)-OH (SEQ ID NO: 61)
Ac-D-Lys-(hCys-Thr-D-Phe-Arg-Trp-Pen)-NH₂ (SEQ ID NO: 62)
H-D-Lys-(hCys-Thr-D-Phe-Arg-Trp-Pen)-NH₂ (SEQ ID NO: 63)
Ac-D-Lys-(hCys-Thr-D-Phe-Arg-Trp-Pen)-NH₂ (SEQ ID NO: 64) or
H-D-Lys-(hCys-Thr-D-Phe-Arg-Trp-Pen)-NH₂ (SEQ ID NO: 65).

EC-50 and Selectivity Ratios for exemplary compounds of the invention are provided below:
001554C Ac-Arg-(hCys-Asn-D-Phe-Arg-Trp-Pen)-NH₂ (SEQ ID NO: 1)
001555C Ac-Arg-(hCys-Gln-D-Phe-Arg-Trp-Pen)-NH₂ (SEQ ID NO: 2)
001556C Ac-Arg-(hCys-Ser-D-Phe-Arg-Trp-Pen)-NH₂ (SEQ ID NO: 3)
001574C: Ac-Arg-(hCys-Thr-D-Phe-Arg-Trp-Pen)-NH₂ (SEQ ID NO: 4)
001576C: Ac-Arg-(Glu-Gln-D-Phe-Arg-Trp-Apr)-NH₂ (SEQ ID NO: 5)

Administration of a compound or pharmaceutically acceptable salt thereof or a composition comprising a compound or pharmaceutical salt of a compound of the invention useful to practice the methods described herein, can be continuous, hourly, four times daily, three time daily, twice daily, once daily, once every other day, twice weekly, once weekly, once every two weeks, once a month, or once every two months, or longer or some other intermittent dosing regimen.

Examples of administration of a compound or composition comprising a compound or pharmaceutical salt of a compound of the invention include peripheral administration. Examples of peripheral administration include oral, subcutaneous, intraperitoneal, intramuscular, intravenous, rectal, transdermal or intranasal forms of administration.

As used herein, peripheral administration can include all forms of administration of a compound or a composition comprising a compound of the instant invention which excludes intracranial administration. Examples of peripheral administration include, but are not limited to, oral, parenteral (e.g., intramuscular, intraperitoneal, intravenous or subcutaneous injection, extended release, slow release implant, depot and the like), nasal, vaginal, rectal, sublingual or topical routes of administration, including transdermal patch applications and the like.

The nomenclature used to define the peptides is that typically used in the art wherein the amino group at the N-terminus appears to the left and the carboxyl group at the C-terminus appears to the right. Where the amino acid has D and L isomeric forms, it is the L form of the amino acid that is represented unless otherwise explicitly indicated.

The compounds of the invention useful for practicing the methods described herein may possess one or more chiral centers and so exist in a number of stereoisomeric forms. All stereoisomers and mixtures thereof are included in the scope of the present invention. Racemic compounds may either be separated using preparative HPLC and a column with a chiral stationary phase or resolved to yield individual enantiomers utilizing methods known to those skilled in the art. In addition, chiral intermediate compounds may be resolved and used to prepare chiral compounds of the invention.

The compounds described herein may exist in one or more tautomeric forms. All tautomers and mixtures thereof are included in the scope of the present invention. For example, a claim to 2-hydroxypyridinyl would also cover its tautomeric form, α-pyridonyl.

Unless defined otherwise, all technical and scientific terms used herein have the same meaning as commonly understood by one of ordinary skill in the art to which this invention belongs. Also, all publications, patent applications, patents and other references mentioned herein are incorporated by reference in their entirety.

| Symbol | Meaning |
|---|---|
| Ac | acyl group |
| hCys | Homocysteine |
| Dpr | 2,3-Diaminopropionic acid |
| Dbu | 2,4-Diaminobutyric acid |
| Orn | Ornithine |

Unless otherwise indicated, with the exception of the N-terminal amino acid, all abbreviations (e.g. Ala) of amino acids in this disclosure stand for the structure of -NH-C(R)(R')-CO-, wherein R and R' each is, independently, hydrogen or the side chain of an amino acid (e.g., R=CH₃ and R'=H for Ala), or R and R' may be joined to form a ring system.

### Pharmaceutical compositions/Administration

In accordance with any method or composition described herein, in embodiments, provided herein is a unit dosage of a MC4R agonist described herein. In embodiments, the unit dosage contains 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, or 2 mg of the agonist. In embodiments, the unit dosage is suitable for injection, e.g., subcutaneous injection. In embodiments, the unit dosage is disposed in a delivery device suitable for injection, e.g., subcutaneous injection. In embodiments, the unit dosage is disposed in a syringe suitable for injection, e.g., subcutaneous injection, or a pen-type injector. Exemplary pen-type injectors are described, e.g., in US 8512297B2, US5688251A, US5820602A, US2014/0163526A1, and US5226895A, incorporated herein by reference.

In embodiments, also provided herein is a pharmaceutical composition comprising a MC4R agonist described herein. In embodiments, the pharmaceutical composition includes a therapeutically effective amount of a MC4R agonist described herein. A therapeutically effective amount of the agonist can vary according to factors such as the disease state, age, sex, and weight of the individual, and the ability of the agonist to elicit a desired response in the individual, *e.g.,* amelioration of at least one disorder parameter, *e.g.,* a parameter of obesity or hyperphagia, or amelioration of at least one symptom of the disorder, *e.g.*, obesity, hyperphagia, or Prader Willi Syndrome (PWS). In embodiments, a therapeutically effective amount is also one in which any toxic or a detrimental effect of the composition is outweighed by the therapeutically beneficial effects.

In certain embodiments, the agonist may be prepared with a carrier that will protect it against rapid release, such as a controlled release formulation, including implants, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known. See, *e.g*., Sustained and Controlled Release Drug Delivery Systems, J. R. Robinson, ed., Marcel Dekker, Inc., New York, 1978.

In other embodiments, the agonist can be prepared using methods described in WO2014/144842, incorporated herein by reference. In embodiments, the agonist is prepared in a formulation comprising an anionic excipient, e.g., PEG-carboxylic acid, fatty acid having 10 or more carbon atoms, and/or anionic phospholipid. In embodiments, the anionic phospholipid is described in WO2014/144842 (e.g., at pages 7-9). In some embodiments, the anionic phospholipid is 1,2-distearoyl-sn-Glycero-3-Phosphoethanolamine (DSPE), optionally conjugated to polyethylene glycol (PEG), the structure of which is: 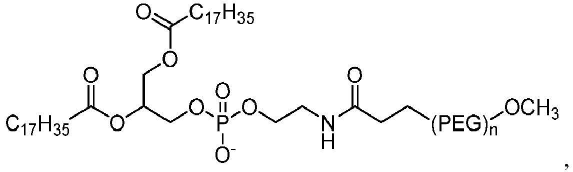 with the value of "n" varying with molecular weight. In embodiments, the fatty acid is described in WO2014/144842 (e.g., at page 9). In embodiments, the PEG-carboxylic acid is described in WO2014/144842 (e.g., at pages 9-11). In embodiments, the molar ratio of the agonist to the anionic excipient ranges from about 1:1 to about 1:10.

In embodiments, the agonist forms an ionic complex with the other components of the formulation, and e.g., provides a desirable pharmacokinetic profile for the agonist (e.g., extend duration of drug action and/or minimize adverse effects). In embodiments, the formulation is a sustained release formulation. In embodiments, the formulation provides reduced fluctuations in concentration of the agonist after administration.

A MC4R agonist described herein, can be administered to a subject, e.g., human subject, by various methods. In embodiments, the route of administration is one of: intravenous injection or infusion, subcutaneous injection, or intramuscular injection. In embodiments, the route of administration is subcutaneous injection.

In embodiments, pharmaceutical compositions, e.g., comprising a MC4R agonist described herein, can be administered with medical devices. For example, compositions comprising the agonist can be administered with a needleless hypodermic injection device, such as the devices disclosed in U.S. Pat. No. 5,399,163, 5,383,851, 5,312,335, 5,064,413, 4,941,880, 4,790,824, or 4,596,556. Examples of implants and modules include: U.S. Pat. No. 4,487,603, which discloses an implantable micro-infusion pump for dispensing medication at a controlled rate; U.S. Pat. No. 4,486,194, which discloses a therapeutic device for administering medicaments through the skin; U.S. Pat. No. 4,447,233, which discloses a medication infusion pump for delivering medication at a precise infusion rate; U.S. Pat. No. 4,447,224, which discloses a variable flow implantable infusion apparatus for continuous drug delivery; U.S. Pat. No. 4,439,196, which discloses an osmotic drug delivery system having multi-chamber compartments; and U.S. Pat. No. 4,475,196, which discloses an osmotic drug delivery system. Other such implants, delivery systems, and modules can also be used.

In embodiments, continuous administration can be indicated, e.g., via subcutaneous pump. In embodiments, the agonist is administered via a syringe (e.g., prefilled syringe), an implantable device, a needleless hypodermic injection device, an infusion pump (e.g., implantable infusion pump), or an osmotic delivery system.

In embodiments, the agonist is administered at a unit dosage, e.g., comprising 0.1-10 mg, e.g., 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.5, 3, 3.5, 4, 4.5, 5, 5.5, 6, 6.5, 7, 7.5, 8, 8.5, 9, 9.5, or 10 mg of the agonist, e.g., subcutaneously.

In embodiments, the agonist is administered in a bolus at a dose of between 0.1-10 mg, e.g., 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.5, 3, 3.5, 4, 4.5, 5, 5.5, 6, 6.5, 7, 7.5, 8, 8.5, 9, 9.5, or 10 mg of the agonist, e.g., subcutaneously.

In embodiments, the agonist is administered continuously, e.g., via a pump, e.g., subcutaneous pump.

In embodiments, the agonist, e.g., a unit dosage of the agonist, is disposed within a delivery device, e.g., a syringe (e.g., prefilled syringe), an implantable device, a needleless hypodermic injection device, an infusion pump (e.g., implantable infusion pump), or an osmotic delivery system.

In embodiments, a daily dosage of the agonist is administered, e.g., subcutaneously, to a subject. In embodiments, the daily dosage of the agonist is about 0.1 mg to about 10 mg, e.g., 0.1-0.2, 0.2-0.4, 0.4-0.6, 0.6-0.8, 0.8-1, 1-1.2, 1.2-1.5, 1.5-2, 2-2.5, 2.5-3, 3-3.5, 3.5-4, 4-4.5, 4.5-5, 5-5.5, 5.5-6, 6-6.5, 6.5-7, 7-7.5, 7.5-8, 8-8.5, 8.5-9, 9-9.5, 9.5-10 mg, e.g., administered subcutaneously.

In embodiments, the agonist, is administered, e.g., via one or multiple administrations, over a period of at least 3 weeks, e.g., at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 weeks or more, or at least 1, 2, 3, 4, 5, 6, 7, 8, 8, 9, 10, 11, or 12 months or more, or at least 1, 2, 3, 4 years or more. In embodiments, where multiple administrations are provided of the agonist, the time interval in between any two of the administrations is at least 6 hours, e.g., 6 h, 12 h, 24 h, 1 day, 2 days, 3 days, 4 days, 5 days, 6 days, 7 days, 1 week, 2 weeks, 3 weeks, 4 weeks, or more. In embodiments, the interval in between any two of the administrations is 1 day.

### Kits

A MC4R agonist described herein can be provided in a kit. The kit includes a MC4R agonist described herein and, optionally, a container, a pharmaceutically acceptable carrier and/or informational material. The informational material can be descriptive, instructional, marketing or other material that relates to the methods described herein and/or the use of the agonist for the methods described herein.

The informational material of the kits is not limited in its form. In one embodiment, the informational material can include information about production of the agonist, physical properties of the agonist, concentration, date of expiration, batch or production site information, and so forth. In one embodiment, the informational material relates to methods for administering the agonist, *e.g*., by a route of administration described herein and/or at a dose and/or dosing schedule described herein.

In one embodiment, the informational material can include instructions to administer an agonist described herein in a suitable manner to perform the methods described herein, *e.g.,* in a suitable dose, dosage form, or mode of administration (*e.g.,* a dose, dosage form, or mode of administration described herein). In another embodiment, the informational material can include instructions to administer an agonist to a suitable subject, *e.g.,* a human, *e.g.,* an obese human, e.g., severely obese human.

The informational material of the kits is not limited in its form. In many cases, the informational material, *e.g.,* instructions, is provided in printed matter, *e.g.,* a printed text, drawing, and/or photograph, *e.g.,* a label or printed sheet. However, the informational material can also be provided in other formats, such as Braille, computer readable material, video recording, or audio recording. In another embodiment, the informational material of the kit is contact information, *e.g*., a physical address, email address, website, or telephone number, where a user of the kit can obtain substantive information about an agonist described herein and/or its use in the methods described herein. The informational material can also be provided in any combination of formats.

In addition to an agonist, the composition of the kit can include other ingredients, such as a surfactant, a lyoprotectant or stabilizer, an antioxidant, an antibacterial agent, a bulking agent, a chelating agent, an inert gas, a tonicity agent and/or a viscosity agent, a solvent or buffer, a stabilizer, a preservative, a pharmaceutically acceptable carrier and/or a second agent for treating a condition or disorder described herein. Alternatively, the other ingredients can be included in the kit, but in different compositions or containers than an agonist described herein.

In some embodiments, a component of the kit is stored in a sealed vial, *e.g*., with a rubber or silicone closure (*e.g.,* a polybutadiene or polyisoprene closure). In some embodiments, a component of the kit is stored under inert conditions (*e.g*., under Nitrogen or another inert gas such as Argon). In some embodiments, a component of the kit is stored under anhydrous conditions (*e.g*., with a desiccant). In some embodiments, a component of the kit is stored in a light blocking container such as an amber vial.

An agonist described herein can be provided in any form, *e.g*., liquid, frozen, dried or lyophilized form. It is preferred that a composition including the agonist described herein be substantially pure and/or sterile. When an agonist described herein is provided in a liquid solution, the liquid solution preferably is an aqueous solution, with a sterile aqueous solution being preferred. In one embodiment, the agonist is supplied with a diluents or instructions for dilution. The diluent can include for example, a salt or saline solution, *e.g.*, a sodium chloride solution having a pH between 6 and 9, lactated Ringer's injection solution, D5W, or PLASMA-LYTE A Injection pH 7.4^{®} (Baxter, Deerfield, IL).

The kit can include one or more containers for the composition containing an agonist described herein. In some embodiments, the kit contains separate containers, dividers or compartments for the composition and informational material. For example, the composition can be contained in a bottle, vial, IV admixture bag, IV infusion set, piggyback set or syringe (e.g., prefilled syringe), and the informational material can be contained in a plastic sleeve or packet. In other embodiments, the separate elements of the kit are contained within a single, undivided container. For example, the composition is contained in a bottle, vial or syringe that has attached thereto the informational material in the form of a label. In embodiments, the composition is contained in an injector device, e.g., a pen-type injector. The containers of the kits can be air tight, waterproof (*e.g*., impermeable to changes in moisture or evaporation), and/or light-tight.

### EXAMPLES

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the compounds of the present invention and practice the claimed methods. The following working examples specifically point out various aspects of the present invention, and are not to be construed as limiting in any way the remainder of the disclosure.

### Example 1: Synthesis of Polypeptides of Formula (I and II)

The polypeptides of Formula (I and II) were prepared by conventional solid phase peptide synthesis. The peptide chain was elongated in a step-wise manner starting with its C-terminal end amino acid derivative coupled on to a suitably selected solid support resin known to be suitable for peptide synthesis. For the synthesis of peptide with a C-terminal amide function, Rink amide MBHA resin was employed as solid support. For the synthesis of peptides with the C-terminal free carboxyl function, resins such as 2-chlorotrityl chloride resin, Wang, or Merrifield resin may be utilized that form an ester bond with the Fmoc-amino acid. Most of these ester linked Fmoc-amino acid-resin types are commercially available from various sources and generally used when feasible.

### Synthesis of Disulfide-cyclized Peptides

The linear derivative of a disulfide cyclic peptides amide was assembled using Fmoc chemistry on a solid-phase peptide synthesizer. The Fmoc-Rink amide resin was placed in a reaction vessel and swollen with NMP. It was then treated with 20% piperidine in NMP for 15 minutes, followed by 3 washes of NMP. The resin was tested for positive Kaiser's test (Kaiser, E., Colescot, R. L., Bossinge, C. D. & Cook, P. I. Anal. Biochem., 1990, 34: 595-598 ). It was resuspended in NMP and mixed with the required first C-terminal Fmoc-amino acid derivative and HOBt. The coupling reaction was started by the addition of HBTU reagent and DIPEA. After mixing for 2-3 hours, the completion of coupling was confirmed by a negative Kaiser's test on a small aliquot of the resin withdrawn from the reaction mixture. The resin was then washed three times with NMP. Thereafter, the Fmoc group was removed as described earlier and the whole cycle repeated with the second C-terminal Fmoc-amino acid derivative as described. The same cycle of reactions was repeated sequentially with each of the incoming amino acid. In the case of peptides with an N-terminal acetyl group, the Fmoc deprotected peptide resin was treated for 10 minutes with acetic anhydride and pyridine. The resin after testing negative for Kaiser's test was washed with NMP, dichlromethane and dried *in vacuo.* The Fmoc-amino acid derivatives were used for the synthesis of these peptides. The trifunctional amino acid derivatives used were the following: Fmoc-Cys(Trt)-OH, Fmoc-Trp(Boc)-OH, Fmoc-Arg(Pbf)-OH, Fmoc-Asn(Trt)-OH, Fmoc-Gln(Trt)-OH, Fmoc-hCys(Trt)-OH, Fmoc-Pen(Trt)-OH, Fmoc-Glu(OBut)-OH, and Fmoc-D-Phe-OH.

To cleave the peptide off the resin as well as deprotect the side chain functions, the peptide resin was taken in 2% TIS/5% water/5% (w/v) DTT/88% TFA. The solution was allowed to mix for 3.5 hours and then filtered. The filtrate was mixed with cold anhydrous ethyl ether. The precipitate was collected by centrifugation. The solvent was decanted and the peptide pellet was re-suspended in fresh ether. The ether workup was repeated two more times. The peptide was dried *in vacuo.* The crude linear peptide product was diluted to a concentration of 2 mg/mL in 5% acetic acid and 0.5M iodine/methanol was added dropwise with vigorous stirring until a persistent pale yellow color of the solution was achieved. The solution was stirred for additional 10 minutes. Excess iodine was then quenched by adding 1M sodium thiosulfate under mixing until the mixture was rendered colorless. The cyclized peptide solution was lyophilized and the crude powder purified by preparative HPLC using a reversed-phase C-18 column. The purified product fractions were pooled and lyophilized. The peptides were analyzed by mass spectrometry using electrospray ionization technique and identified to correct mass.

### Synthesis of Lactam-cyclized Peptides

The cyclic lactam peptides were also synthesized by standard solid phase peptide synthesis methods. For peptides with a C-terminus Dpr, an Fmoc-Dpr(Mtt)-BHA and for peptides with a C-terminal Abu, an Fmoc-Abu(Mtt)-BHA resin was transferred to a solid phase peptide synthesizer reactor. The Fmoc group, was removed as described above and the next Fmoc-protected amino acid, such as for example Fmoc-Trp(Boc)-OH, was coupled to the resin through standard coupling procedures. The Fmoc protective group was removed and the remaining amino acids added individually in the correct sequence, by repeating coupling and deprotection procedures until the amino acid sequence was completed. For glutamic acid or aspartic acid coupling Fmoc-Glu(OPip) or Fmoc-Asp(OPip) was employed. The fully assembled peptide was then acetylated at the N-terminus as per method described earlier for the disulfide series of peptides. The orthogonally protected side chains were then removed. For example, a peptide resin with either an orthogonally protected side chain of Glu as 2-phenylisoproply (OPip) ester or Dpr as 4-methyltrityl (Mtt), were cleaved by treatment with 1% TFA in dicholoromethane. The deprotected peptide resin was suspended in NMP, and treated with HBTU/DIPEA. After cyclization (a negative Kaiser's test), the peptide-resin was washed with DCM and dried. The cyclic peptide was cleaved from the resin along with any remaining protective groups using trifluoroacetic acid (TFA) in the presence of water and 1,2-ethanedithiol (EDT). The product was collected by precipitation upon the addition of cold anhydrous ether and collected by centrifugation. Final purification was by reversed phase HPLC using a reversed phase C-18 column. The purified peptide collected by lyophilization and analyzed for its mass by mass spectrometry using electron spray methodology.

One skilled in the art can modify the synthesized polypeptides of Formula (I and II) described herein.

### Radioligand Binding Assays:

Receptor binding assays for determining the binding constant (K_{d}) or inhibition concentration (IC₅₀) for displacing a radio-labeled ligand from the receptor of a cyclic peptide of Formula (I or II) may be conducted by any means known in the art.

As an example, the cell membrane preparations for a binding assay are prepared from CHO-K1 cells transfected to stably express hMC receptor subtypes 1, 3, 4 or 5. Competitive inhibition of [¹²⁵I](Tyr²)-(Nle⁴-D-Phe⁷)- alpha-MSH ([¹²⁵I]-NDP-α-MSH binding is carried out in polypropylene 96 well plates. Briefly, the cell membranes (1-10 µg protein/well), prepared as described above, is incubated in 50 mM Tris-HCl at pH 7.4 containing 0.2% BSA, 5 mM MgCl₂, 1 mM CaCl₂ and 0.1 mg/mL bacitracin, with increasing concentrations of the test compound and 0.1-0.3 nM [¹²⁵I]-NDP-α-MSH for approximately 120 minutes at 37 °C. Bound [¹²⁵I]-NDP-α-MSH ligand is separated from free [¹²⁵I]-NDP-α- MSH by filtration through GF/C glass fiber filter plates (Unifilter^{®}, Meriden, CT, USA) presoaked with 0.1 % (w/v) polyethylenimine (PEI). Filters are washed three times with 50 mM Tris-HCl at pH 7.4 at a temperature of approximately 0-4 °C and then assayed for radioactivity. The binding data are analyzed by computer-assisted non-linear regression analysis.

### Cyclic AMP Stimulation Assay

Functional assays to determine agonist or antagonist status of a cyclic peptide of polypeptide of Formula (I and II) are performed using methods known in the art.

### Electrochemiluminescence (ECL) Assay

Stimulation of intracellular cyclic AMP (cAMP) levels by the peptides is determined in a dose dependent manner by an electrochemiluminescence (ECL) assay (Meso Scale Discovery, Gaithersburg, MD, USA; referred to hereinafter as "MSD"). Briefly, the CHO-K1 cells stably expressing the hMC receptor subtypes are suspended in RMPI 1640^{®} assay buffer (RMPI 1640 buffer contains 0.5 mM IBMX, and 0.2% protein cocktail (MSD blocker A)). About 7,000 cells/well of the transgenic CHO-K1 cells stably expressing hMC receptor subtypes 1, 3, 4 or 5 are dispensed in 384-well Multi-Array plates (MSD) containing integrated carbon electrodes and coated with anti-cAMP antibody. Increasing concentrations of the test compounds are added and the cells are incubated for approximately 40 minutes at 37 °C. A cell lysis buffer (HEPES-buffered saline solution with MgCl₂ and Triton X-100^{®} at pH 7.3) containing 0.2% protein cocktail and 2.5 nM TAG^{™} ruthenium-labeled cAMP (MSD) is added and the cells are incubated for approximately 90 minutes at room temperature. At the end of the second incubation period, the read buffer (Tris-buffered solution containing an ECL co-reactant and Triton X- 100 at pH 7.8) is added and the cAMP levels in the cell lysates are immediately determined by ECL detection with a Sector Imager 6000 reader^{®} (MSD). Data are analyzed using a computer-assisted non-linear regression analysis (XL fit; IDBS) and reported as either an EC50 value. The EC50 represents the concentration of an agonist compound needed to obtain 50% of the maximum reaction response, e.g., 50% of the maximum level of cAMP as determined using the assay described above.

### cAMP Measurement Assay

Human MC4-R transfected cells are grown to confluence in 96 well plates (plating approximately 250,000 cells per well). The cells are treated in triplicate sets with 0.2 mM isobutylmethylxanthine (IBMX) and graded concentrations of the peptide or alternatively the peptide in the presence of 20 nM NDP-MSH. Cells similarly treated but with only 20 nM NDP-MSH serve as positive controls in a volume of 200 µL. A buffer blank serving as a negative control is also included. After incubation of one hour at 37 °C, the cells are lysed by the addition of 50 µL of a cell lysis buffer. Total cAMP accumulated in 250 µL of this incubation medium is quantitated using a commercially available low pH cAMP assay kit (Amersham BioSciences) as per procedure specified by the kit supplier. A peptide showing cAMP accumulation in the range same or higher than the alpha-MSH as positive control is considered to be an agonist. The data for agonist is plotted and curve fitted to determine the EC50 value. A peptide showing accumulation in the same range as the negative control (buffer blank in the absence of alpha-MSH) is ineffective at the test concentration. A peptide showing attenuated accumulation is considered to be an antagonist if there is inhibition in cAMP when alpha-MSH is also present in the assay. Similar assay can be performed with hMC-1R, hMC-3R, and hMC-5R cells.

### cAMP Accumulation Measurement via a β-galactosidase (β-Gal) Reporter System

A chemiluminescence readout system that uses an enzyme fragment complementation (EFC) system with β-galactosidase (β-Gal) as the functional reporter system was used. This assay system for various melanocortin receptor systems is commercially available (cAMP Hunter GPCR assay system, Discoverx Corp, Fremont, CA). This assay utilizes the β-Gal enzyme that is split into two complementary portions; EA for Enzyme Acceptor and ED for Enzyme Donor. In the assay, the ED portion fused to cAMP is made to compete with cAMP generated by cells for binding to a cAMP-specific antibody. The EA is then added to form active β-Gal with any unbound ED-cAMP. This active enzyme then converts a chemiluminescent substrate to generate an output signal that is recored on a standard microplate reader.

Briefly, 10000 cells per well are plated overnight and each well (cells incubated with 10 µl assay buffer) is then incubated with 4X serial concentration of the test compound in the cell assay buffer (5 µL) and cAMP antibody reagent (5 µL) for 30 min at 37 °C. The cell lysis buffer (20 µL) containing ED-cAMP coupled enzyme fragment and the reporter substrate (Emerald II-Galacton Star, 5:1) is then added and incubated for 60 min at room temperature. Next, 20 µL of EA β-Gal fragment reagent is added. After further incubation for 120 min at room temperature, the chemiluminescence is measured by a plate reader (Envision).

The EC-50 data for examplary polypeptides of structural formulas I and II are shown in below.

### Example: EC50 values and selectivity.

| **Compound ID** | **EC-50 (nM)** | | | | **Selectivity Ratios** | | |
|---|---|---|---|---|---|---|---|
| | **MC-1 R** | **MC-3R** | **MC-4R** | **MC-5R** | **MC4R / MC1R** | **MC4R / MC3R** | **MC4R / MC5R** |
| 001554C SEQ ID NO:1 | 235.06 | >3000 | **1.60** | 735.47 | **146.75** | **> 1872.97** | 459.17 |
| 001555C SEQ ID NO:2 | 47.49 | 0.52 | **0.19** | 124.33 | **244.35** | 2.68 | 639.81 |
| 001556C SEQ ID NO:3 | 228.52 | >3000 | **1.86** | 1701.86 | **123.06** | **>1615.55** | 916.48 |
| 001574C SEQ ID NO:4 | 516.38 | >3000 | **1.11** | 2047.30 | **465.77** | **>2705.95** | 1846.64 |
| 001576C SEQ ID NO:5 | 23.59 | 1.16 | **0.66** | 67.26 | **35.57** | 1.75 | 101.41 |
| | | | | | | | |
| RM-493 | 1.80 | 1.06 | **0.94** | 1684.02 | **1.92** | 1.13 | 1799.06 |
| Melanotan II | 0.28 | 1.33 | 3.08 | 80.48 | 0.09 | 0.43 | 26.11 |

### EQUIVALENTS

The disclosures of each and every patent, patent application, and publication cited herein are hereby incorporated herein by reference in their entirety. While this invention has been disclosed with reference to specific aspects, it is apparent that other aspects and variations of this invention may be devised by others skilled in the art without departing from the true spirit and scope of the invention. The appended claims are intended to be construed to include all such aspects and equivalent variations.

### NUMBERED EMBODIMENTS:

1. A compound of formula I: 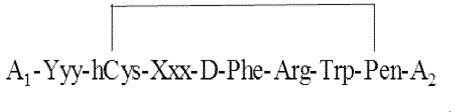
   where Xxx is Asn, Gin, Ser, or Thr,
   where A₁ is H or Ac,
   where A₂ is OH or NH₂, and
   where Yyy is Lys, Arg, D-Lys, or D-Arg, or
   a pharmaceutically acceptable salt, single stereoisomer, mixture of stereoisomers, ester, tautomer or prodrug thereof.
2. The compound of embodiment 1 wherein the compound is a compound of Formula II. 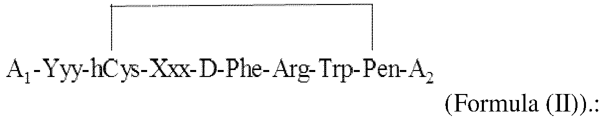
   where Xxx is Asn, Gin, Ser, or Thr,
   where A₁ is H or Ac,
   where A₂ is OH or NH₂, and
   where Yyy is Lys, Arg, D-Lys, or D-Arg.
3. The compound of formula 1 wherein the compound comprises the sequence of any of SEQ ID NOs:1-64.
4. A pharmaceutical composition comprising a compound of any of embodiments 1-3 and a pharmaceutically acceptable carrier.
5. A unit dosage any of the compounds of embodiments 1-3, wherein the unit dosage contains 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 20, 2.5, 3, 3.5, 4, 4.5, 5, 5.5, 6, 6.5, 7, 7.5, 8, 8.5, 9, 9.5, 10, 15, 20, 25, 30, 35, 40, 45, 50 or 100 mg of the compound.
6. The unit dosage of embodiment 5, which contains 0.5 mg, 1.0 mg, or 1.5 mg of the compound.
7. The unit dosage of any of embodiments 5-6, suitable for injection, e.g., subcutaneous injection.
8. The unit dosage of any of embodiments 5-7, disposed in a delivery device suitable for injection, e.g., subcutaneous injection.
9. A method of treating a condition or disorder in a subject in need thereof, comprising administering a therapeutically effective amount of an MC4R agonist of any of embodiments 1-3.
10. The method of embodiment 9, comprising eliciting an agonist effect from a melanocortin receptor in the subject.
11. The method of embodiment 9, wherein administering the MC4R agonist elicits an agonist effect from the melanocortin receptor in the subject.
12. The method of embodiment 9, wherein the disorder is a metabolic disease or medical condition accompanied by weight gain such as obesity, feeding disorders or Prader-Willi Syndrome.
13. The method of embodiment 9, wherein the MC4R agonist is administered in an amount sufficient to decrease food intake, decrease body weight, or both, in the subject.
14. The method of embodiment 9, wherein the MC4R agonist is administered in an amount sufficient to decrease appetite in the subject without compromising body weight.
15. The method of embodiment 9, wherein the MC4R agonist is administered in amount sufficient to ameliorate or improve a clinical symptom of obesity, type-II diabetes, a pre-diabetic condition, a blood level of hemoglobin A1C (HblAc) above 6%, hyperinsulimenia, hyperlipidemia, insulin insensitivity, or glucose intolerance; aspects of metabolic syndrome, delaying, inhibiting or preventing the progression of obesity and/or obesity related indications; or partially or totally delaying, inhibiting or preventing the onset or development of obesity or a obesity related indication.
16. The method of embodiment 9, wherein the condition or disorder is obesity or a disorder associated with obesity, e.g., Prader-Willi Syndrome, decreasing food intake in a subject in need thereof, or decreasing body weight.
17. The method of any of the preceding embodiments, comprising administering the agonist in a unit dosage suitable for injection, e.g., subcutaneous injection, to the subject.
18. The method of embodiment 6, wherein the unit dosage is disposed within a delivery device, e.g., a syringe (e.g., prefilled syringe),an implantable device, a needleless hypodermic injection device, an infusion pump (e.g., implantable infusion pump), or an osmotic delivery system.
19. The method of any of the preceding embodiments, wherein the agonist is administered subcutaneously, e.g., by subcutaneous injection.
20. The method of any of the preceding embodiments, wherein the agonist is administered daily over a period of at least 3 weeks, e.g., at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 weeks or more, or at least 1, 2, 3, 4, 5, 6, 7, 8, 9, 10, 11, or 12 months or more, or at least 1, 2, 3, 4 years or more.
21. The method of any of the preceding embodiments wherein administration of the compound decreases body weight in the subject.
22. The method of any of the preceding embodiments, wherein the subject is obese, e.g., severely obese.
23. The method of any of the preceding embodiments, wherein the subject has early onset severe obesity.
24. The method of any of the preceding embodiments, wherein the subject is hyperphagic.
25. The method of any of the preceding embodiments, wherein the subject has a body mass index (BMI) greater than 25 kg/m² (e.g., ≥25, 30, 31, 32, 33, 34, 35, 36, 37, 38, 39, 40, 41, 42, 43, 44, 45, 46, 47, 48, 49, 50 kg/m² or greater) prior to administration of the agonist, e.g., at the time the agonist is prescribed, or at the time of the first administration.
26. The method of any of the preceding embodiments, wherein the subject has failed one or more previous therapies, e.g., exercise, diet, bariatric surgery or vertical banded gastroplasty, or behavioral therapies, prior to administration of the agonist, e.g., at the time the agonist is prescribed, or at the time of the first administration.
27. The method of any of the preceding embodiments, wherein administration of the agonist results in a reduction of food intake by the subject compared to a control (e.g., the food intake of the subject prior to treatment), e.g., wherein the food intake is daily food intake over a period of 24 hours, or one week.
28. The method of any of the preceding embodiments, wherein the agonist comprises the
   sequence 001554C Ac-Arg-(hCys-Asn-D-Phe-Arg-Trp-Pen)-NH₂ (SEQ ID NO: 1)
   001555C Ac-Arg-(hCys-Gln-D-Phe-Arg-Trp-Pen)-NH₂ (SEQ ID NO: 2)
   001556C Ac-Arg-(hCys-Ser-D-Phe-Arg-Trp-Pen)-NH₂ (SEQ ID NO: 3)
   001574C: Ac-Arg-(hCys-Thr-D-Phe-Arg-Trp-Pen)-NH₂(SEQ ID NO: 4)
   001576C: Ac-Arg-(Glu-Gln-D-Phe-Arg-Trp-Apr)-NH₂ (SEQ ID NO: 5)
   Ac-Arg-(hCys-Asn-D-Phe-Arg-Trp-Pen)-OH (SEQ ID NO: 6)
   H-Arg-(hCys-Asn-D-Phe-Arg-Trp-Pen)-NH₂ (SEQ ID NO: 7)
   H-Arg-(hCys-Asn-D-Phe-Arg-Trp-Pen)-OH (SEQ ID NO: 8)
   Ac-D-Arg-(hCys-Asn-D-Phe-Arg-Trp-Pen)-NH₂ (SEQ ID NO: 9)
   H-D-Arg-(hCys-Asn-D-Phe-Arg-Trp-Pen)-NH₂ (SEQ ID NO: 10)
   Ac-D-Arg-(hCys-Asn-D-Phe-Arg-Trp-Pen)-OH (SEQ ID NO:11)
   H-D-Arg-(hCys-Asn-D-Phe-Arg-Trp-Pen)-OH (SEQ ID NO: 12)
   Ac-Lys-(hCys-Asn-D-Phe-Arg-Trp-Pen)-NH₂ (SEQ ID NO: 13)
   Ac-Lys-(hCys-Asn-D-Phe-Arg-Trp-Pen)-OH(SEQ ID NO: 14)
   H-Lys-(hCys-Asn-D-Phe-Arg-Trp-Pen)-NH₂ (SEQ ID NO:15)
   H-Lys-(hCys-Asn-D-Phe-Arg-Trp-Pen)-OH (SEQ ID NO: 16)
   Ac-D-Lys-(hCys-Asn-D-Phe-Arg-Trp-Pen)-NH₂ (SEQ ID NO: 17)
   H-D-Lys-(hCys-Asn-D-Phe-Arg-Trp-Pen)-NH₂ (SEQ ID NO: 18)
   Ac-D-Lys-(hCys-Asn-D-Phe-Arg-Trp-Pen)-OH (SEQ ID NO: 19)
   H-D-Lys-(hCys-Asn-D-Phe-Arg-Trp-Pen)-OH (SEQ ID NO: 20)
   Ac-Arg-(hCys-Gln-D-Phe-Arg-Trp-Pen)-OH (SEQ ID NO: 21)
   H-Arg-(hCys-Gln-D-Phe-Arg-Trp-Pen)-NH₂ (SEQ ID NO: 22)
   H-Arg-(hCys-Gln-D-Phe-Arg-Trp-Pen)-OH (SEQ ID NO: 23)
   Ac-D-Arg-(hCys-Gln-D-Phe-Arg-Trp-Pen)-NH₂ (SEQ ID NO: 24)
   H-D-Arg-(hCys-Gln-D-Phe-Arg-Trp-Pen)-NH₂ (SEQ ID NO: 25)
   Ac-D-Arg-(hCys-Gln-D-Phe-Arg-Trp-Pen)-OH (SEQ ID NO: 26)
   H-D-Arg-(hCys-Gln-D-Phe-Arg-Trp-Pen)-OH (SEQ ID NO: 27)
   Ac-Lys-(hCys-Gln-D-Phe-Arg-Trp-Pen)-NH₂ (SEQ ID NO: 28)
   Ac-Lys-(hCys-Gln-D-Phe-Arg-Trp-Pen)-OH (SEQ ID NO: 29)
   H-Lys-(hCys-Gln-D-Phe-Arg-Trp-Pen)-NH₂ (SEQ ID NO: 30)
   H-Lys-(hCys-Gln-D-Phe-Arg-Trp-Pen)-OH (SEQ ID NO: 31)
   Ac-D-Lys-(hCys-Gln-D-Phe-Arg-Trp-Pen)-NH₂ (SEQ ID NO: 32)
   H-D-Lys-(hCys-Gln-D-Phe-Arg-Trp-Pen)-NH₂ (SEQ ID NO: 33)
   Ac-D-Lys-(hCys-Gln-D-Phe-Arg-Trp-Pen)-OH (SEQ ID NO: 34)
   H-D-Lys-(hCys-Gln-D-Phe-Arg-Trp-Pen)-OH (SEQ ID NO: 35)
   Ac-Arg-(hCys-Ser-D-Phe-Arg-Trp-Pen)-OH (SEQ ID NO: 36)
   H-Arg-(hCys-Ser-D-Phe-Arg-Trp-Pen)-NH₂ (SEQ ID NO: 37)
   H-Arg-(hCys-Ser-D-Phe-Arg-Trp-Pen)-OH (SEQ ID NO: 38)
   Ac-D-Arg-(hCys-Ser-D-Phe-Arg-Trp-Pen)-NH₂ (SEQ ID NO: 39)
   H-D-Arg-(hCys-Ser-D-Phe-Arg-Trp-Pen)-NH₂ (SEQ ID NO: 40)
   Ac-D-Arg-(hCys-Ser-D-Phe-Arg-Trp-Pen)-OH (SEQ ID NO:41)
   H-D-Arg-(hCys-Ser-D-Phe-Arg-Trp-Pen)-OH (SEQ ID NO:42)
   Ac-Lys-(hCys-Ser-D-Phe-Arg-Trp-Pen)-NH₂ (SEQ ID NO: 43)
   Ac-Lys-(hCys-Ser-D-Phe-Arg-Trp-Pen)-OH (SEQ ID NO:44)
   H-Lys-(hCys-Ser-D-Phe-Arg-Trp-Pen)-NH₂ (SEQ ID NO: 45)
   H-Lys-(hCys-Ser-D-Phe-Arg-Trp-Pen)-OH (SEQ ID NO: 46)
   Ac-D-Lys-(hCys-Ser-D-Phe-Arg-Trp-Pen)-NH₂ (SEQ ID NO: 47)
   H-D-Lys-(hCys-Ser-D-Phe-Arg-Trp-Pen)-NH₂ (SEQ ID NO: 48)
   Ac-D-Lys-(hCys-Ser-D-Phe-Arg-Trp-Pen)-OH (SEQ ID NO: 49)
   H-D-Lys-(hCys-Ser-D-Phe-Arg-Trp-Pen)-OH (SEQ ID NO: 50)
   Ac-Arg-(hCys-Thr-D-Phe-Arg-Trp-Pen)-OH (SEQ ID NO: 51)
   H-Arg-(hCys-Thr-D-Phe-Arg-Trp-Pen)-NH₂ (SEQ ID NO: 52)
   H-Arg-(hCys-Thr-D-Phe-Arg-Trp-Pen)-OH (SEQ ID NO: 53)
   Ac-D-Arg-(hCys-Thr-D-Phe-Arg-Trp-Pen)-NH₂ (SEQ ID NO: 54)
   H-D-Arg-(hCys-Thr-D-Phe-Arg-Trp-Pen)-NH₂ (SEQ ID NO: 55)
   Ac-D-Arg-(hCys-Thr-D-Phe-Arg-Trp-Pen)-OH (SEQ ID NO: 56)
   H-D-Arg-(hCys-Thr-D-Phe-Arg-Trp-Pen)-OH (SEQ ID NO: 57)
   Ac-Lys-(hCys-Thr-D-Phe-Arg-Trp-Pen)-NH₂ (SEQ ID NO: 58)
   Ac-Lys-(hCys-Thr-D-Phe-Arg-Trp-Pen)-OH (SEQ ID NO: 59)
   H-Lys-(hCys-Thr-D-Phe-Arg-Trp-Pen)-NH₂ (SEQ ID NO: 60)
   H-Lys-(hCys-Thr-D-Phe-Arg-Trp-Pen)-OH (SEQ ID NO: 61)
   Ac-D-Lys-(hCys-Thr-D-Phe-Arg-Trp-Pen)-NH₂ (SEQ ID NO: 62)
   H-D-Lys-(hCys-Thr-D-Phe-Arg-Trp-Pen)-NH₂ (SEQ ID NO: 63)
   Ac-D-Lys-(hCys-Thr-D-Phe-Arg-Trp-Pen)-NH₂ (SEQ ID NO: 64) or
   H-D-Lys-(hCys-Thr-D-Phe-Arg-Trp-Pen)-NH₂ (SEQ ID NO: 65).

## Claims

1. A compound of Formula I: 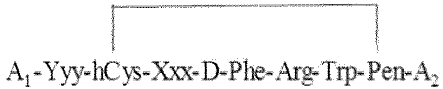
where Xxx is Asn, Gln, Ser, or Thr,
where A₁ is H or Ac,
where A₂ is OH or NH₂, and
where Yyy is Lys, Arg, D-Lys, or D-Arg, or
a pharmaceutically acceptable salt, single stereoisomer, mixture of stereoisomers, or ester thereof.

2. The compound of claim 1, wherein the compound is a compound of Formula II: 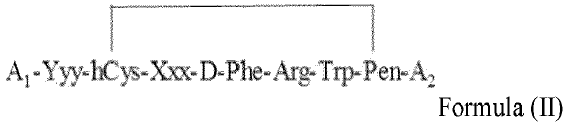
where Xxx is Asn, Gln, Ser, or Thr,
where A₁ is H or Ac,
where A₂ is OH or NH₂, and
where Yyy is Lys, Arg, D-Lys, or D-Arg.

3. The compound of any of claim 1 or 2, wherein the compound comprises the sequence of:
Ac-Arg-(hCys-Asn-D-Phe-Arg-Trp-Pen)-NH₂ (SEQ ID NO: 1)
Ac-Arg-(hCys-Gln-D-Phe-Arg-Trp-Pen)-NH₂ (SEQ ID NO: 2)
Ac-Arg-(hCys-Ser-D-Phe-Arg-Trp-Pen)-NH₂ (SEQ ID NO: 3)
Ac-Arg-(hCys-Thr-D-Phe-Arg-Trp-Pen)-NH₂ (SEQ ID NO: 4)
Ac-Arg-(Glu-Gln-D-Phe-Arg-Trp-Apr)-NH₂ (SEQ ID NO: 5)
Ac-Arg-(hCys-Asn-D-Phe-Arg-Trp-Pen)-OH (SEQ ID NO: 6)
H-Arg-(hCys-Asn-D-Phe-Arg-Trp-Pen)-NH₂ (SEQ ID NO: 7)
H-Arg-(hCys-Asn-D-Phe-Arg-Trp-Pen)-OH (SEQ ID NO: 8)
Ac-D-Arg-(hCys-Asn-D-Phe-Arg-Trp-Pen)-NH₂ (SEQ ID NO: 9)
H-D-Arg-(hCys-Asn-D-Phe-Arg-Trp-Pen)-NH₂ (SEQ ID NO: 10)
Ac-D-Arg-(hCys-Asn-D-Phe-Arg-Trp-Pen)-OH (SEQ ID NO:11)
H-D-Arg-(hCys-Asn-D-Phe-Arg-Trp-Pen)-OH (SEQ ID NO: 12)
Ac-Lys-(hCys-Asn-D-Phe-Arg-Trp-Pen)-NH₂ (SEQ ID NO: 13)
Ac-Lys-(hCys-Asn-D-Phe-Arg-Trp-Pen)-OH (SEQ ID NO: 14)
H-Lys-(hCys-Asn-D-Phe-Arg-Trp-Pen)-NH₂ (SEQ ID NO:15)
H-Lys-(hCys-Asn-D-Phe-Arg-Trp-Pen)-OH (SEQ ID NO: 16)
Ac-D-Lys-(hCys-Asn-D-Phe-Arg-Trp-Pen)-NH₂ (SEQ ID NO: 17)
H-D-Lys-(hCys-Asn-D-Phe-Arg-Trp-Pen)-NH₂ (SEQ ID NO: 18)
Ac-D-Lys-(hCys-Asn-D-Phe-Arg-Trp-Pen)-OH (SEQ ID NO: 19)
H-D-Lys-(hCys-Asn-D-Phe-Arg-Trp-Pen)-OH (SEQ ID NO: 20)
Ac-Arg-(hCys-Gln-D-Phe-Arg-Trp-Pen)-OH (SEQ ID NO: 21)
H-Arg-(hCys-Gln-D-Phe-Arg-Trp-Pen)-NH₂ (SEQ ID NO: 22)
H-Arg-(hCys-Gln-D-Phe-Arg-Trp-Pen)-OH (SEQ ID NO: 23)
Ac-D-Arg-(hCys-Gln-D-Phe-Arg-Trp-Pen)-NH₂ (SEQ ID NO: 24)
H-D-Arg-(hCys-Gln-D-Phe-Arg-Trp-Pen)-NH₂ (SEQ ID NO: 25)
Ac-D-Arg-(hCys-Gln-D-Phe-Arg-Trp-Pen)-OH (SEQ ID NO: 26)
H-D-Arg-(hCys-Gln-D-Phe-Arg-Trp-Pen)-OH (SEQ ID NO: 27)
Ac-Lys-(hCys-Gln-D-Phe-Arg-Trp-Pen)-NH₂ (SEQ ID NO: 28)
Ac-Lys-(hCys-Gln-D-Phe-Arg-Trp-Pen)-OH (SEQ ID NO: 29)
H-Lys-(hCys-Gln-D-Phe-Arg-Trp-Pen)-NH₂ (SEQ ID NO: 30)
H-Lys-(hCys-Gln-D-Phe-Arg-Trp-Pen)-OH (SEQ ID NO: 31)
Ac-D-Lys-(hCys-Gln-D-Phe-Arg-Trp-Pen)-NH₂ (SEQ ID NO: 32)
H-D-Lys-(hCys-Gln-D-Phe-Arg-Trp-Pen)-NH₂ (SEQ ID NO: 33)
Ac-D-Lys-(hCys-Gln-D-Phe-Arg-Trp-Pen)-OH (SEQ ID NO: 34)
H-D-Lys-(hCys-Gln-D-Phe-Arg-Trp-Pen)-OH (SEQ ID NO: 35)
Ac-Arg-(hCys-Ser-D-Phe-Arg-Trp-Pen)-OH (SEQ ID NO: 36)
H-Arg-(hCys-Ser-D-Phe-Arg-Trp-Pen)-NH₂ (SEQ ID NO: 37)
H-Arg-(hCys-Ser-D-Phe-Arg-Trp-Pen)-OH (SEQ ID NO: 38)
Ac-D-Arg-(hCys-Ser-D-Phe-Arg-Trp-Pen)-NH₂ (SEQ ID NO: 39)
H-D-Arg-(hCys-Ser-D-Phe-Arg-Trp-Pen)-NH₂ (SEQ ID NO: 40)
Ac-D-Arg-(hCys-Ser-D-Phe-Arg-Trp-Pen)-OH (SEQ ID NO:41)
H-D-Arg-(hCys-Ser-D-Phe-Arg-Trp-Pen)-OH (SEQ ID NO:42)
Ac-Lys-(hCys-Ser-D-Phe-Arg-Trp-Pen)-NH₂ (SEQ ID NO: 43)
Ac-Lys-(hCys-Ser-D-Phe-Arg-Trp-Pen)-OH (SEQ ID NO:44)
H-Lys-(hCys-Ser-D-Phe-Arg-Trp-Pen)-NH₂ (SEQ ID NO: 45)
H-Lys-(hCys-Ser-D-Phe-Arg-Trp-Pen)-OH (SEQ ID NO: 46)
Ac-D-Lys-(hCys-Ser-D-Phe-Arg-Trp-Pen)-NH₂ (SEQ ID NO: 47)
H-D-Lys-(hCys-Ser-D-Phe-Arg-Trp-Pen)-NH₂ (SEQ ID NO: 48)
Ac-D-Lys-(hCys-Ser-D-Phe-Arg-Trp-Pen)-OH (SEQ ID NO: 49)
H-D-Lys-(hCys-Ser-D-Phe-Arg-Trp-Pen)-OH (SEQ ID NO: 50)
Ac-Arg-(hCys-Thr-D-Phe-Arg-Trp-Pen)-OH (SEQ ID NO: 51)
H-Arg-(hCys-Thr-D-Phe-Arg-Trp-Pen)-NH₂ (SEQ ID NO: 52)
H-Arg-(hCys-Thr-D-Phe-Arg-Trp-Pen)-OH (SEQ ID NO: 53)
Ac-D-Arg-(hCys-Thr-D-Phe-Arg-Trp-Pen)-NH₂ (SEQ ID NO: 54)
H-D-Arg-(hCys-Thr-D-Phe-Arg-Trp-Pen)-NH₂ (SEQ ID NO: 55)
Ac-D-Arg-(hCys-Thr-D-Phe-Arg-Trp-Pen)-OH (SEQ ID NO: 56)
H-D-Arg-(hCys-Thr-D-Phe-Arg-Trp-Pen)-OH (SEQ ID NO: 57)
Ac-Lys-(hCys-Thr-D-Phe-Arg-Trp-Pen)-NH₂ (SEQ ID NO: 58)
Ac-Lys-(hCys-Thr-D-Phe-Arg-Trp-Pen)-OH (SEQ ID NO: 59)
H-Lys-(hCys-Thr-D-Phe-Arg-Trp-Pen)-NH₂ (SEQ ID NO: 60)
H-Lys-(hCys-Thr-D-Phe-Arg-Trp-Pen)-OH (SEQ ID NO: 61)
Ac-D-Lys-(hCys-Thr-D-Phe-Arg-Trp-Pen)-NH₂ (SEQ ID NO: 62)
H-D-Lys-(hCys-Thr-D-Phe-Arg-Trp-Pen)-NH₂ (SEQ ID NO: 63)
Ac-D-Lys-(hCys-Thr-D-Phe-Arg-Trp-Pen)-NH₂ (SEQ ID NO: 64)
H-D-Lys-(hCys-Thr-D-Phe-Arg-Trp-Pen)-NH₂ (SEQ ID NO: 65),
or a pharmaceutically acceptable salt thereof.

4. A pharmaceutical composition comprising a compound of any of claims 1-3 and a pharmaceutically acceptable carrier.

5. A unit dosage comprising a compound of any of claims 1-3, wherein the unit dosage contains 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2.0, 2.5, 3, 3.5, 4, 4.5, 5, 5.5, 6, 6.5, 7, 7.5, 8, 8.5, 9, 9.5, 10, 15, 20, 25, 30, 35, 40, 45, 50 or 100 mg of the compound.

6. The unit dosage of claim 5, which contains 0.5 mg, 1.0 mg, or 1.5 mg of the compound.

7. The unit dosage of any of claims 5-6, suitable for injection, e.g., subcutaneous injection.

8. The unit dosage of any of claims 5-7, disposed in a delivery device suitable for injection, e.g., subcutaneous injection.

9. A composition for use in treating a condition or disorder in a subject in need thereof, comprising administering a therapeutically effective amount of a compound of any of claims 1-3, a pharmaceutical composition of claim 4, or a unit dosage of any of claims 5-8.

10. The composition for use of claim 9, comprising eliciting an agonist effect from a melanocortin receptor in the subject.

11. The composition for use of claim 9, wherein the condition or disorder is a metabolic disease or medical condition accompanied by weight gain such as obesity, a feeding disorder, or Prader-Willi Syndrome.

12. The composition for use of claim 9, wherein the compound, pharmaceutical composition, or unit dosage is administered in an amount sufficient to decrease food intake, decrease body weight, or both, in the subject.

13. The composition for use of claim 9, wherein the compound, pharmaceutical composition, or unit dosage is administered in amount sufficient to:
(i) ameliorate or improve a clinical symptom of obesity, type-II diabetes, a prediabetic condition, a blood level of hemoglobin A1C (Hb1Ac) above 6%, hyperinsulimenia, hyperlipidemia, insulin insensitivity, glucose intolerance, or aspects of metabolic syndrome;
(ii) delay, inhibit, or prevent the progression of obesity and/or obesity related indications; or
(iii) partially or totally delay, inhibit, or prevent the onset or development of obesity or an obesity related indication.

14. The composition for use of claim 9, wherein:
(a) the condition or disorder is obesity or a disorder associated with obesity, e.g., Prader-Willi Syndrome; or
(b) the compound, pharmaceutical composition, or unit dosage is administered daily over a period of at least 3 weeks, e.g., at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 weeks or more, or at least 1, 2, 3, 4, 5, 6, 7, 8, 9, 10, 11, or 12 months or more, or at least 1, 2, 3, 4 years or more.

15. The composition for use of claim 9, wherein the subject:
(i) is obese, e.g., severely obese;
(ii) has early onset severe obesity;
(iii) is hyperphagic;
(iv) has a body mass index (BMI) greater than 25 kg/m² (e.g., ≥25, 30, 31, 32, 33, 34, 35, 36, 37, 38, 39, 40, 41, 42, 43, 44, 45, 46, 47, 48, 49, 50 kg/m² or greater) prior to administration of the compound, pharmaceutical composition, or unit dosage, e.g., at the time the compound, pharmaceutical composition, or unit dosage is prescribed, or at the time of the first administration of the compound, pharmaceutical composition, or unit dosage; and/or
(v) has failed one or more previous therapies, e.g., exercise, diet, bariatric surgery or vertical banded gastroplasty, or behavioral therapies, prior to administration of the compound, pharmaceutical composition, or unit dosage, e.g., at the time the compound, pharmaceutical composition, or unit dosage is prescribed, or at the time of the first administration of the compound, pharmaceutical composition, or unit dosage.
